(19) **Europäisches Patentamt**
**European Patent Office**
**Office européen des brevets**

(11) **EP 4 061 214 B1**

(12) **EUROPEAN PATENT SPECIFICATION**

(45) Date of publication and mention
of the grant of the patent:
**09.07.2025 Bulletin 2025/28**

(21) Application number: **19809416.1**

(22) Date of filing: **21.11.2019**

(51) International Patent Classification (IPC):
*A61B 5/107* (2006.01)   *A61B 5/11* (2006.01)
*G06T 7/00* (2017.01)   *G16H 50/00* (2018.01)
*A61B 5/00* (2006.01)   *G06T 7/73* (2017.01)
*G06T 7/80* (2017.01)   *G16H 50/20* (2018.01)
*G16H 30/40* (2018.01)

(52) Cooperative Patent Classification (CPC):
**A61B 5/1127; A61B 5/1079; G06T 7/73;**
**G06T 7/80; G16H 30/40; G16H 50/20;**
A61B 5/4561; A61B 2560/0233; A61B 2560/0406;
G06T 2207/30204

(86) International application number:
**PCT/EP2019/082111**

(87) International publication number:
**WO 2021/098964 (27.05.2021 Gazette 2021/21)**

(54) **METHOD FOR OBTAINING A SPATIAL PATTERN OF AN ANATOMICAL STRUCTURE OF A SUBJECT AND RELATED SYSTEM**

VERFAHREN ZUM ERHALTEN EINES RAUMMUSTERS EINER ANATOMISCHEN STRUKTUR EINES PROBANDEN UND ZUGEHÖRIGES SYSTEM

PROCÉDÉ D'OBTENTION D'UN MOTIF SPATIAL D'UNE STRUCTURE ANATOMIQUE D'UN SUJET ET SYSTÈME ASSOCIÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**28.09.2022 Bulletin 2022/39**

(73) Proprietor: **Banyan Technologies AG**
**9500 Villach (AT)**

(72) Inventors:
• **AGUZZI, Damiano**
**05100 Terni TR (IT)**

• **SALEPPICO, Juri**
**05035 Narni TR (IT)**

(74) Representative: **Porta & Consulenti Associati**
**S.p.A.**
**Via Winckelmann, 1**
**20146 Milano (IT)**

(56) References cited:
WO-A1-2007/005011   WO-A2-2011/149182
WO-A2-2018/081136   US-A1- 2003 181 830
US-A1- 2017 303 859   US-B2- 7 095 886

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The present invention relates to a method for obtaining a spatial pattern of an anatomical structure of a subject. The invention further relates to a system configured to carry out such methods and to related markers.

Background of the invention

Biometric and morphological analysis of biological structures are topics of great interest in biomechanical research and in a variety of clinical fields.

The possibility of analysing the morphology and to extract biometric data, such as geometric and dimensional data, relating to biological structures of the human or animal body first appeared with the introduction of medical imaging techniques, which allowed to reveal internal structures hidden by the skin or by other organs.

The extraction of biometric data plays an important role for example in the fields of traumatology and orthopaedic, in appliances for preventing, detecting and monitoring postural disorders or bone deformities, or also in the morphological analysis of a subject's biological structure, for example for research aims.

In recent decades especially spinal and postural functional assessment has progressively gained importance for clinical practice as a result of both financial and clinical issues.

Epidemiological studies recognize that postural defects frequently involve concurrent diseases that may become manifest years after the onset of the first symptoms, and may further degenerate into more serious pathologic conditions even in the absence of previous trauma or genetic predisposition. For these reasons, it would be extremely useful an early detection of postural defects even when no advanced symptoms are observed.

Postural diseases are also frequently caused by stability issues due to unbalance of the neuromuscular tone. Especially in this case, a continuous monitoring of results is important for a positive outcome of related treatments.

Morphologic and biometric analysis of biological structures is conventionally carried out on radiographic or tomographic images. However, these imaging techniques do not constitute an optimal choice for screening or routine monitoring exams, because frequent exposure to ionising radiations might be harmful for the patients.

Related art

Methods and systems for obtaining spatial patterns of biological structures that do not involve ionising radiations are known in the art.

In particular, among different alternatives, stereophotogrammetry is emerging as a robust non-contacting measurement technique that allows to estimate three-dimensional coordinates of points on an object/subject based on measurements made in a series of photographic images taken from different angles.

EP 3 225 155 A1 discloses a system and method for detecting and monitoring postural diseases in a patient, based on photographic images of the patient carrying position reflectors in predefined positions.

The system comprises a computer, a digital image capturing device (such as a camera or a webcam) and a coaxial illumination system, position reflectors (also indicated as luminous tags) applied on the patient, a suitable software for elaboration and data acquisition, and a platform.

The method comprises the steps of positioning luminous tags at predefined locations of the body of the patient; connecting via a USB port the digital image capturing device (camera or webcam) to the computer; acquiring images or photograms; elaborating the images through the software; printing and/or copying data on a hardware support.

The position reflectors consist of simple reflecting bands attached, at predefined locations, onto the skin of the patient by means of an adhesive.

The document further describes that the software allows, through an automatic search of the position of the reflectors within the image of the patient, to reproduce in 3D the spinal curve of the patient and to analyse it for detecting possible defects. The software comprises an algorithm for video elaboration, for position search in the image, for fitting a diagram and for storing the data analysed.

D'amico et al. (D'Amico M, Kinel E, Roncoletta P (2017) Normative 3D opto-electronic stereophotogrammetric posture and spine morphology data in young healthy adult population, PLoS ONE 12(6): e0179619. https://doi.org/10.1371/journal.pone.0179619) presents an observational study in which posture of a cohort of young adults was analyzed using a stereophotogrammetric opto-electronic system and related method.

The system comprises a set of at least two, up to 16 cameras, a set of 27 passive markers to be applied on the patient at specific relevant body landmarks such as along the spine, a baropodometric platform to support the patient in standing position and a computer.

A basic configuration of the system comprises at least two cameras located behind the patient, differently angled, and markers positioned on the posterior side of the patient. In more advanced configurations, markers can be applied also on the anterior side of the patient and cameras on the front side can be added.

Images of the patient carrying the markers are acquired. The cameras are sensitive to the wavelength corresponding to the brightness level of the markers, therefore the acquired images depict the distribution of the markers within a calibrated space over a dark background. Further relevant prior art documents are WO 2011/149182 A2 and US 2003/181830 A1.

Summary of the invention

The Applicant has observed that the system disclosed by D'amico et al. is quite complex as its primary application is motion analysis. It requires a high number of markers and an increasing number of cameras in order to obtain a higher precision. Such a system requires in turn a very complex processing algorithm that may result in slow and inefficient in static postural analysis applications.

In particular, the Applicant observed that the use of a plurality of cameras positioned at different angles with respect to the subject and further having their focal axis inclined with respect to a horizontal direction involves a complex image calibration, since the relative reference systems of all cameras must be mapped to a common absolute (calibrated) reference system by means of lengthy mathematical operations.

The Applicant has found that at least some of the issues mentioned above may be overcome by acquiring calibrated images of a plurality of markers having a known three-dimensional shape, such markers being applied on respective body landmarks of an anatomical structure of a human or animal subject.

In this way, calibrated images may be obtained having an informational content that allows to accurately determine, through suitable mathematical algorithms, the position of contact points of the markers with the related body landmarks.

Within the framework of the present description and in the following claims, the expression "contact point of the marker with the body landmark" or similar expressions are meant to indicate a point located substantially at the geometrical centre of a contact surface of the marker with the skin of the subject. This contact point substantially corresponds, when the marker is applied with a sufficient precision on the subject, to the position of the relevant body landmark onto which the marker is meant to be applied.

The Applicant has also found that thanks to the informational content of the calibrated images, a three-dimensional pattern of the anatomical structure of interest may be accurately determined, based on which parameters of interest related to the anatomical structure of the subject can be subsequently extracted.

The Applicant also found that the calibrated images of the markers having a three-dimensional shape may be obtained by a simple system which can make use of even a single digital image capturing device and a low number of markers, without the need to expose the subject to any harmful ionising radiation.

Accordingly, in a first aspect, the invention relates to a method according to claim 1 for obtaining a spatial pattern of an anatomical structure of a subject, comprising the steps of:

a) acquiring, from at least one digital image capturing device, at least one uncalibrated image of a calibration reference applied on a surface configured to receive the subject, said calibration reference having at least one known dimension and defining at least one known direction;
b) defining an absolute calibrated reference system of coordinates based on the calibration reference depicted in said at least one uncalibrated image; and
c) acquiring, from said at least one digital image capturing device, at least a first and at least a second calibrated image of a plurality of markers applied on a corresponding plurality of body landmarks of the anatomical structure of the subject at respective contact points with the body landmarks, said plurality of markers being arranged within said absolute calibrated reference system;

wherein said at least a first calibrated image depicts a marker spatial arrangement in a first plane and said at least a second calibrated image depicts the marker spatial arrangement in a second plane different from the first plane, the first and the second plane being representative of a relative orientation of the plurality of markers with respect to the at least one digital image capturing device;

wherein said markers of said plurality of markers each comprise a respective three-dimensional main body shaped as a polyhedron of N faces and having N-1 exposed faces arranged in a known geometric relationship with the contact point of the marker with the respective body landmark; and

wherein at least one of said N-1 exposed faces of each marker is identifiable in said at least first and second calibrated images by means of an image recognition algorithm.

Within the framework of the present description and in the following claims, the expression "uncalibrated image" is used, in contrast with the expression "calibrated image", to indicate an image which has not undergone any image calibration operation yet.

Within the framework of the present description and in the following claims, by the expression "surface configured to receive the subject", a surface parallel to or coinciding with the ground is meant to be indicated, said surface extending at least to include a vertical projection of the subject positioned thereon when the first and second calibrated images are captured.

Advantageously, the method according to the invention allows to obtain calibrated images of markers applied on an anatomical structure of interest that are "enhanced" by an informational content related to the spatial arrangement of contact points of the markers with the respective body landmarks.

Such informational content can in particular be further employed, by means of suitable geometric operations, to "map" the anatomical structure of interest, by extracting the position of the contact points with the respective body landmarks, as later detailed.

The calibrated images are captured by means of a digital image capturing device, therefore avoiding both the exposure of the subject to the harmful ionising radiations of conventional radiographic examinations, as well as the consequent negative environmental impact due to the disposal of related radioactive waste materials.

The method according to the present invention can advantageously be carried out by using, in a minimum configuration, a single digital image capturing device, the two calibrated images being captured by changing a relative orientation of the subject with respect to the digital image capturing device.

Moreover, the method according to the present invention is simple and fast as only two images are needed, captured in two perpendicular planes, namely two planes sharing a "vertical" axial coordinate, as will be explained in more detail hereinafter.

Furthermore, the number of markers required to carry out the method is advantageously minimized.

These advantageous effects are in particular conferred by the three-dimensional configuration of the markers.

Indeed, the three-dimensional main body of the markers is advantageously visible when the markers are applied on the subject even when the region of the body on which the markers are applied is "angled" with respect to the digital image capturing device - for example when the subject has markers applied on the back and an image is captured from a "side" of the subject in standing position - as the markers project from the body and, if made of proper dimension, are not hidden by protruding structures of the body such as, for example, the scapulae.

In particular, the main body of the markers has a polyhedral structure in which exposed faces are in a known geometric relationship with the contact point of the marker with the corresponding body landmark. These geometric properties confer the relevant informational content based on which the contact point of the marker with the respective body landmark can be determined, by running an *ad hoc* algorithm, even when such point is hidden in the image.

Moreover, data obtained from the above method can advantageously be further used to evaluate postural issues, such as for example scoliosis, lordosis or other polymorphisms, related for example to algic diseases, such as cervical pain, lumbago, sciatica, knee pain, coxalgia, back pain, bursitis, or to degenerative diseases, such as arthrosis, meniscal diseased, ligament or tendon diseases, contractures ..., or yet post-surgical diseases due to knee, foot, hip or spinal surgery. Other possible advantageous applications of the method are for example the extraction of measurements of body structures, preferably skeletal structures, such as for example long bones (e.g. femur, tibia).

According to a second aspect, the present invention relates to a system according to claim 8 for obtaining a spatial pattern of an anatomical structure of a subject, said system comprising:

-    a calibration reference applied on a surface configured to receive the subject, said calibration reference having at least one known dimension and defining at least one known direction;

-    a plurality of three-dimensional markers configured to be applied on a corresponding plurality of body landmarks of the anatomical structure of the subject at respective contact points, each marker comprising a respective three-dimensional main body, shaped as a polyhedron of N faces, and having N-1 exposed faces arranged in a known geometric relationship with the contact point of the marker with the respective body landmark,

wherein at least one of said N-1 exposed faces of each marker is identifiable, by means of an image recognition algorithm, in an image of the marker captured by a digital image capturing device; and

-    at least one digital image capturing device configured to:

(i) capture at least one uncalibrated image of said calibration reference; and

(ii) capture at least a first and at least a second calibrated image of said plurality of markers applied on said corresponding plurality of body landmarks and arranged within said absolute calibrated reference system defined based on said at least one uncalibrated image of the calibration reference,

wherein said at least a first calibrated image depicts a marker spatial arrangement in a first plane and said at least a second calibrated image depicts the marker spatial arrangement in a second plane different from the first plane, the first and second planes being representative of a relative orientation of the plurality of markers with respect to the at least one digital image capturing device.

Advantageously, the system according to the present invention is particularly simple as it only requires a plurality of markers having the particular shape described above, at least one suitably programmed processor, and at least one digital image capturing device.

As stated above in connection with the method, in a minimum configuration the system includes a single digital image capturing device, which captures two images by changing the relative orientation of the subject with respect to the digital image capturing device. In this way, complex and lengthy setting and adjusting operations, needed when different image capturing devices are used - as in the systems of the prior art - are advantageously avoided.

Moreover, the system according to the invention requires a minimized number of markers to capture and acquire the "enhanced" calibrated images the anatomical structure of the subject.

According to a third aspect, the present invention relates to a system according to claim 8, wherein each marker is configured to obtain a spatial pattern of an anatomical structure of a subject, said marker being configured to be applied on a body landmark of the anatomical structure of the subject at a respective contact point and comprising a three-dimensional main body shaped as a polyhedron of N faces, having N-1 exposed faces arranged in a known geometric relationship with the contact point of the marker with the respective body landmark;

wherein at least one of said N-1 exposed faces of the marker is identifiable, by means of an image recognition algorithm, in an image of the marker captured by a digital image capturing device.

As stated above, the three-dimensional body of the marker is advantageously visible when the marker is applied on the subject even when the region of the body on which the marker is applied is "angled" with respect to the digital image capturing device - for example when the subject has the marker applied on the back and an image is captured from a "side" of the subject in standing position - as the marker is configured to project from the body.

Moreover, the main body of the marker has a polyhedral structure with faces in a known geometric relationship with a contact point of the marker with the corresponding body landmark, therefore processable by a suitable *ad hoc* algorithm to trace back the position of such a contact point even when such point is hidden in the image.

In a preferred configuration, the marker is therefore advantageously a small, light object of a hard material, such as for example plastic, that can be manufactured according to any suitable manufacturing process.

The present invention can have, in one or more of the above aspects thereof, one or more of the preferred features described hereinafter, which can be combined with one another as desired depending on the application requirements.

Within the framework of the present description and in the following claims, all numerical values indicating amounts, parameters, percentages and so on are always to be intended as preceded by the term "about", if not otherwise stated. Moreover, all numerical value ranges include all possible combinations of the maximum and minimum numerical values and all possible intermediate ranges, besides those specifically indicated below. The endpoints of the disclosed range are included unless indicated otherwise.

The method further comprises the step of:

d) determining for each marker of said plurality of markers a position of the contact point with the respective body landmark of the subject within said absolute calibrated reference system.

The positions of the contact points of markers so obtained, also overall indicated, sometimes, as "pattern of points", can advantageously be used, by means of further elaboration, to reconstruct a shape of the anatomical structure and to extract parameters of interest of such structure.

In embodiments, said steps a)-d) are executed by a same processor.

In this way, the overall method can be carried out by a same processor belonging to a same computational system or device. Therefore, both the preliminary calibration operations and the determination of the position of markers can be carried out on-site, during the image capturing session, or the determination of the position of markers can also be carried out on-site soon after.

In alternative embodiments, said steps a)-c) are executed by a first processor, and said step d) is executed by a second processor, said second processor optionally being a remote processor with respect to said first processor.

In this case, the determination of the position of the contact points of the markers can be carried out by a processor belonging to a different (or even remote) computational system with respect to the first processor on which the preliminary calibration has been executed during the image capturing session. For example, the first processor can be a "local" processor, located in proximity of the location in which image capturing is carried out. The term "remote" is meant to indicate, in the present description and in the attached claims, a processor located physically removed in space from the first processor, such as in another room, building or even city or country.

Therefore, according to this embodiment of the invention the elaboration to determine the pattern of points can be deferred in time and carried out in different locations, and can be advantageously executed on a more performing computational device or system. In turn, the first "local" processor can be implemented by a simplified system requiring less computational power, such as for example a personal computer or a tablet.

The system preferably further comprises:

- a processor programmed to:

  (i) acquire said at least one uncalibrated image of the calibration reference from the at least one digital image capturing device;
  (ii) define said absolute calibrated reference system of coordinates based on said at least one uncalibrated image;
  (iii) acquire said at least a first and said at least a second calibrated images from the at least one digital image capturing device; and
  (iv) determine for each marker of said plurality of markers a position of the contact point with the respective body landmark of the subject within said absolute calibrated reference system.

In this case, the system requires a single processor, that can be embodied e.g. by a same computational device located on-site, at the location where image capturing is carried out, or elsewhere and suitably connected to the digital image capturing device.

In alternative embodiments, the system preferably further comprises:

- a first processor programmed to:

  (i) acquire said at least one uncalibrated image of the calibration reference from the at least one digital image capturing device;
  (ii) define said absolute calibrated reference system of coordinates based on said at least one uncalibrated image;
  (iii) acquire said at least a first and said at least a second calibrated images from the at least one digital image capturing device; and
  (iii_bis) transmit said at least a first and said at least a second calibrated images of said plurality of markers to a second processor;

and

- the second processor programmed to:

  (iii_ter) acquire said at least a first and said at least a second calibrated images of said plurality of markers from said first processor; and

  (iv) determine for each marker of said plurality of markers the position of the contact point with the respective body landmark of the subject within said absolute calibrated reference system,

  said second processor optionally being remote with respect to said first processor.

In this alternative embodiment, the system comprises different processors embodied by computational systems that may be for example located in different places and working in different timeframes. In such case, a first processor executes the calibration operations needed on-site to calibrate the digital image capturing device before capturing at least two calibrated images, whereas the second processor carries out the elaboration operations leading to the determination of the positions of contact point of the marker with the relevant landmarks. For example, the first processor can be located in proximity of the location in which image capturing is carried out, whereas the second processor can be a remote processor located elsewhere.

Preferably, said subject is human.

Preferably, said surface configured to receive the subject receives, in use, the subject in standing position thereon.

Preferably, said anatomical structure of the subject is the spine.

Preferably, said calibration reference is used to calibrate the images, namely to determine a pixel/cm conversion factor needed to extract metric data from the images subsequently captured with the digital image capturing device.

Preferably, the first plane and the second plane are perpendicular to each other.

Preferably, said first and second planes share a common vertical coordinate.

In this way, the determination of the position of the contact points of the markers within the absolute calibrated reference system is particularly simplified, as better detailed hereinafter.

Preferably, said first plurality of body landmarks are located on a posterior side of the subject, on the back of the subject.

Therefore, preferably the first calibrated image depicts a posterior side of the subject.

Preferably said first plane of the first calibrated image substantially coincides with a coronal plane of the subject in standing position on the surface, and said second plane of the second calibrated image substantially coincides with a sagittal plane of the subject in standing position on the surface.

Preferably, after capturing the first calibrated image and before capturing the second calibrated image, the subject is rotated of 90° about a vertical axis.

In this way, the digital image capturing device is not moved and no resetting is required.

Preferably, said calibration reference comprises at least three tags applied onto said surface, a first and a second tag being positioned at a first known distance from one another along a first axis, and said second tag and a third tag being positioned at a second known distance from one another along a second axis perpendicular to said first axis,

wherein said first axis and said second axis are parallel, when the first and second calibrated images are captured by the digital image capturing device, to a latero-lateral direction and, respectively, to a postero-anterior direction of the subject.

Preferably, the calibration operation is carried out based on said known distances between tags and on directions defined by said first and second axes along which tags are positioned.

Moreover, based on the first and second axes so defined, a third axis extending substantially vertically from the reference surface is preferably found by means of known mathematical operations, thereby defining the absolute calibrated reference system centered in the first tag.

Preferably, said first and second distances are equal to each other. In such a case, the calibration operation is thus particularly simplified.

In this case, preferably said first and second distances are comprised between 20 and 60 cm, more preferably they are equal to 42 cm.

Preferably, the surface configured to receive the subject is square-shaped and said first, second and third tags are positioned at three corners thereof.

More preferably, said calibration reference comprises a fourth tag positioned at the fourth corner of the squared surface.

Preferably, the surface configured to receive the subject has a first reference plane substantially coinciding, in use, with a coronal plane of the subject standing thereon, and a second reference plane substantially coinciding, in use, with a sagittal plane of the subject standing thereon.

Said reference planes are preferably orthogonal planes passing from a center of symmetry of the squared surface.

Preferably, said first plane of the first calibrated image is substantially parallel to or coincides with the first reference plane of the surface, and said second plane of the second calibrated image is substantially parallel to or coincides with the second reference plane of the surface.

Said at least one uncalibrated image acquired in step a) is captured by the at least one digital image capturing device with a focal axis thereof substantially aligned to either the first reference plane or the second reference plane of said surface.

Moreover, preferably, said at least a first and said at least a second calibrated image acquired in step c) are captured by the at least one digital image capturing device with the focal axis thereof substantially aligned to the second reference plane, respectively to the first reference plane, of said surface.

In the present description and in the attached claims, by "substantially aligned to a plane", a maximum displacement in a direction transversal to said plane comprised between 0 and 5 mm is meant to be indicated.

This alignment avoids images to be distorted due to being erroneously translated along axis x.

Preferably, said at least one uncalibrated image acquired in step a) and said at least a first and said at least a second calibrated image acquired in step c) are captured by the at least one digital image capturing device with a lens thereof positioned at a same fixed distance along the focal axis from either the first reference plane or the second reference plane of said surface.

The distance between the lens of the camera and the the first, respectively second, reference plane of the surface is chosen based on the lens of the digital image capturing device that is used and on the height of the subject.

The distance between said lens and the first, respectively second, reference plane of the surface is preferably comprised between 235 and 290 cm.

More preferably, said distance between the lens of the camera and the first, respectively second, reference plane of the surface is comprised between 235 and 250 cm.

Preferably, said surface configured to receive the subject is an upper surface of a support rotatably mounted onto a stationary stand configured to be laid on the ground.

Preferably, said support is rotatable between fixed angular positions angularly spaced of 90°.

In such a case, preferably, after capturing the first calibrated image and before capturing the second calibrated image, the subject is rotated of 90° about said vertical axis by rotating the support with the subject standing thereon.

In this way, the occurrence of positional changes of the subject due to the rotation, that might cause macroscopic differences in the two images, are minimized.

In this way, the position, rotation or arrangement of the support can be adjusted as needed before the image capturing step or based on the subject to be analysed.

Preferably, said support comprises a guide configured to set up an angle between feet of the subject positioned thereon.

In this case, said guide preferably comprises an angled spacer having two oblique sides configured to abut, in use, to an inner side of the feet of the subject, said sides diverging from one another moving from a posterior to an anterior side of the support and forming an angle between each other.

Such guide both fixes the angle between the feet and fixes a lateral displacement of the subject with respect to the support.

In the present description and in the attached claims, the terms "anterior", "posterior", "front", "back" and similar will be used with reference to the subject positioned on the surface configured to receive the subject or to upper surface of the above-described support.

In the present description and in the attached claims, the terms "horizontal", "vertical", "lateral" and similar terms will be used with reference to configuration of the system when the same is in use.

Preferably, said angle is comprised between 25° and 35°.

More preferably, said angle is of about 30°.

The standing position with feet spaced apart at 30° is indicated as an optimal position to evaluate postural issues. Moreover, the guide allows the position of the subject to be repeatable between subsequent image capturing steps in a same execution of the method, for example between capturing the first and the second calibrated images, if the subject needs for any reason, in-between, to leave the support.

Preferably, the guide comprises a length limiter comprising an anterior bar and a posterior bar. Such bars advantageously limit the position of the feet of the subject in an anteroposterior direction.

More preferably, a distance between the anterior and the posterior bars can be adjusted so as to adapt to different feet dimensions.

Preferably, the guide aids in correctly positioning the subject at a symmetrically centered position onto the support, so that the coronal plane and the sagittal plane of the subject are respectively substantially parallel to or coincide with median planes of the support.

In the present description and in the attached claims, by "substantially parallel", an angle of 0° ± 1° is meant to be indicated.

Preferably, said first and second reference planes of the surface are median planes of the support.

Preferably, said first and second median planes are planes passing from a centre of symmetry of the upper surface of the support.

Preferably, said at least one uncalibrated image acquired in step a) and said at least a first and said at least a second calibrated image acquired in step c) are captured by the at least one digital image capturing device with the optic axis thereof positioned at a same vertical distance from the surface.

The vertical distance between the focal axis of the camera and the surface configured to receive the subject is selected based on the height of the subject.

Preferably, such vertical distance corresponds to the height of the iliac crests of the subject.

Such vertical distance is preferably comprised between 30 and 130 cm.

Preferably, said at least one uncalibrated image acquired in step a), and said at least a first and said at least a second calibrated image acquired in step c), are captured by the at least one digital image capturing device with the focal axis thereof substantially parallel to said surface.

In this way, distortion of the image is minimized or avoided, and as a consequence measurement errors in the calibrated images subsequently captured are advantageously minimized.

Preferably, said at least a first and said at least a second calibrated image acquired in step c) are captured by the at least one digital image capturing device with the focal axis thereof substantially parallel to the postero-anterior direction, respectively to the latero-lateral direction of the subject.

Preferably, said at least one uncalibrated image acquired in step a) is captured by the at least one digital image capturing device with the focal axis thereof substantially parallel either to the postero-anterior direction or to the latero-lateral direction of the subject.

Preferably, the digital image capturing device is a digital camera.

Preferably, the camera is provided with a commercial lens selected from a 18-55 mm lens and a 24-105 mm lens.

Preferably, said digital image capturing device has a resolution of at least 48 megapixel.

Preferably, said step d) comprises:

d1) determining for each marker of the plurality of markers a first initial position of the respective contact point of the marker in the first calibrated image acquired in step c),

d2) determining for each marker of the plurality of markers a second initial position of the respective contact point of the marker in the second calibrated image acquired in step c), and

d3) combining the first and second initial positions, respectively determined in steps d1) and d2) from said first and second calibrated images, to obtain the position of each marker.

Preferably, said steps d1) and d2) each comprise, for each marker of the plurality of markers depicted in said at least a first, respectively said at least a second, calibrated image:

d4) identifying one or more exposed faces of the marker by recognizing a respective distinctive feature thereof;

d5) determining in said at least a first calibrated image, respectively in said at least a second calibrated image, based on an orientation of the one or more exposed faces of the marker recognized, a position of a center of symmetry of the main body of the marker;

d6) determining in said at least a first calibrated image, respectively in said at least a second calibrated image, based on an orientation of the one or more exposed faces of the marker recognized, the direction of an axis of symmetry of the marker passing through a contact surface of the marker with the respective body landmark;

d7) determining in said at least a first calibrated image, respectively in said at least a second calibrated image, the first initial position, respectively the second initial position, of the contact point of the marker along said axis of symmetry of the marker, at a known distance from the center of symmetry of the main body of the marker.

Preferably, said step d) comprises defining a relative reference system of coordinates for each distinctive feature recognized, said relative reference system of coordinates being centered at a center of symmetry of the distinctive feature recognized.

Preferably, said step d3) comprises averaging a common axial coordinate between said first and said second initial positions respectively determined from said at least a first and said at least a second calibrated images.

Preferably, the methods according to the first, second and third aspects of the invention further comprise the step of:

e) fitting the positions of the contact points of markers of said plurality of markers with a curve.

Preferably, said step e) of fitting the positions of the contact points of markers with a curve is carried out by means of an interpolation operation selected from polynomial interpolation and spline interpolation, preferably by means of cubic spline interpolation.

Preferably, the methods according to the first, second and third aspects of the invention further comprise the step of:

f) calculating, from the curve fitted in step e), parameters of interest related to the anatomical structure of the skeleton of the subject, and/or displaying said curve.

Preferably, said parameters of interest calculated in step f) are selected among distances, areas, angles, curvatures.

Preferably, said parameters of interest calculated in step f) comprise a Cobb angle.

Preferably, said step c) further comprises acquiring a third calibrated image of the marker spatial arrangement on either the first or the second plane, viewed from an opposite direction with respect to the first or second calibrated image, respectively.

By adding a third image, a higher precision in the determination of the contact points of markers with the body landmarks can be achieved.

In such case, said step d) further comprises:

d21) determining for each marker of the plurality of markers a third initial position of the respective contact point of the marker in the third calibrated image acquired in step c);

wherein said steps d21) comprises, for each marker of the plurality of markers depicted in said third calibrated image:

d41) identifying one or more exposed faces of the marker by recognizing a respective distinctive feature thereof,

d51) determining in the third calibrated image, based on an orientation of the one or more exposed faces of the marker recognized, a position of a center of symmetry of the main body of the marker,

d61) determining in the third calibrated image, based on an orientation of the one or more exposed faces of the marker recognized, the direction of an axis of symmetry of the marker passing through a contact surface of the marker with the respective body landmark,

d71) determining in the third calibrated image, the third initial position of the contact point of the marker along said axis of symmetry of the marker, at a known distance from the center of symmetry of the main body of the marker;

wherein step d3) comprises combining the first, the second and the third initial positions, respectively determined in steps d1), d2) and d21) from said at least a first, said at least a second and said third calibrated images, to obtain the position of each marker; and

wherein said step d3) comprises averaging a first common axial coordinate between said first, second and third initial positions respectively determined from said at least a first, said at least a second and said third calibrated images, and averaging a second common axial coordinate between said second and third initial positions.

Preferably, said plurality of markers comprises at least four markers, and said plurality of body landmarks comprises at least:

-    a first body landmark selected among cervical vertebrae C4 and C5;

-    a second body landmark selected among dorsal vertebrae D4, D5, D and D7;

-    a third body landmark selected among dorsal vertebrae D8, D9, D10, D11 and D12;

-    a fourth body landmark selected among lumbar vertebrae L1, L2, L3, L4 and L5.

More preferably, said plurality of markers preferably comprises at least six markers, and said plurality of body landmarks further comprises the left and right acromions.
In other embodiments, said plurality of markers preferably comprises up to twenty markers, said plurality of body landmarks further comprising additional body landmarks preferably selected from: apex of left and right axillary cavities, lower apex of left and right scapulae, left and right posterior superior iliac spines, left and right olecranons, left and right lateral knee, left and right outer malleolus, left and right anterior superior iliac spines, left and right radial styloid processes, left and right medial malleolus.
Preferably, the system further comprises the support as described above, configured to receive the subject in standing position, said support having said calibration reference positioned on an upper surface thereof.
Preferably, the main body of each marker is shaped as a regular polyhedron.
More preferably, the main body of each marker has a shape selected from an octahedron, a dodecahedron, and icosahedron.
In this way, the geometric calculations leading to the definition of the contact point of the marker with the corresponding body landmark are particularly simplified.
Preferably, the main body of each marker is shaped as a dodecahedron.
Preferably, each marker of said plurality of markers comprises a base having a surface configured to contact (also referred herein as "contact surface") a respective body landmark.
In embodiments, said base of the marker corresponds to one of the N faces of the main body of the marker.
In other embodiments, the base comprises a plate fixed to one of the N faces of the marker, having said surface configured to contact the skin of the subject.
Preferably, the base is a plate of a regular shape, preferably circular.
In this case, the contact point of the marker with the respective body landmark corresponds to a centre of symmetry of the regular contact surface, such as for example the centre of the circular contact surface.

In this way, the shape of the base is independent of the shape of the faces of the marker, and the base can be better adapted for attachment to the skin of the subject.

In particularly advantageous embodiments, said base comprises an elongated stem that projects from a proximal face of the marker, and a plate on which the contact surface of the marker is defined.

In the present description and in the attached claims, the terms "proximal" and "distal" are used with reference to the skin of the subject on which, in use, the markers are attached at the respective landmark.

The provision of a connecting stem allows, by suitably choosing a length thereof, to increase a "projecting distance" of the marker, so as to make the marker visible and detectable in images captured by the digital image capturing devices also when it is applied behind particularly protruding structures of the body of the subject, such as for example behind the scapulae.

Preferably, such plate and/or such stem, if present, are integral with the main body of the marker.

Preferably, the marker is attached to the body landmark of the subject by means of a medical grade adhesive selected from an adhesive paste and an adhesive sheet.

Preferably, said adhesive is in the form of a removable adhesive sheet which may be provided on the base of the marker.

Preferably, each exposed face of the marker is provided with a distinctive feature.

Preferably, one or more groups of exposed faces of the marker each share a same distinctive feature.

Preferably, said distinctive feature comprises graphic sign over a background of a contrasting color.

Preferably, said graphic sign is a glyph.

Preferably, said glyphs comprises dark or white squared modules over a contrasting background, or of white modules over a dark background.

Preferably, said glyph is white over a dark background.

In this way, a particularly effective contrast is exerted between the glyph and the background.

Preferably, the marker is made of a light plastic material.

Preferably, the marker is made of a polymeric material selected from acrylonitrile-butadienestyrene (ABS), poly-lactic acid (PLA), high impact polystyrene (HIPS), nylon, carbon fiber, polycarbonate and acrylic styrene acrylonitrile (ASA).

More preferably, the marker is made of ABS.

In preferred embodiments, the marker may be manufactured by 3D printing.

Preferably, a length of an edge of the regular polyhedral main body of the marker is comprised between about 7 and 15 mm.

Preferably, the marker can be inscribed in a sphere having a diameter comprised between 20 and 40 mm.

In the present description and in the attached claims, the expression "inscribe in a sphere" refers to building an imaginary sphere external to the regular polyhedral main body of the marker, such sphere including on its surface all the vertexes of the polyhedron.

Preferably, the circular contact surface of the markers has a diameter comprised between 10 and 30 mm.

Preferably, a distance between the proximal face and the contact surface of the markers is comprised between 0 and 25 mm.

Brief description of the drawings

Additional features and advantages of the present invention will be better illustrated by the following detailed description of some of preferred embodiments thereof, made with reference to the accompanying drawings, in which structural or functional elements having the same or similar function are indicated by identical or similar reference numbers.

In the drawings:

- FIG. 1 illustrates a first preferred embodiment of a marker according to the present invention;

- FIG. 2 illustrates a second preferred embodiment of a marker according to the present invention;

- FIG. 3 illustrates examples of distinctive features of markers according to preferred embodiments of the invention;

- FIGs. 4-5 illustrate an exemplary grid, empty and partially filled, used to form the distinctive features of FIG. 3 according to preferred embodiments of the invention;

- FIG. 6 illustrates an exemplary distribution of the markers on specific body landmarks of an anatomical structure of a human subject;

- FIGs. 7-8 illustrate a system according to a preferred embodiment of the invention, in two different image capturing configurations;

- FIG. 9 illustrates a first embodiment of a support according to a preferred embodiment of the invention;

- FIG. 10 illustrates a second embodiment of a support according to a preferred embodiment of the invention;

- FIG. 11 is a flowchart of a method according to a preferred embodiment of the invention for obtaining a spatial pattern of an anatomical structure of a subject;

- FIG. 12 schematically illustrates the calculation of parameters of interest on the curve fitted according to a method according to a preferred embodiment of the invention;

- FIG. 13 is a flowchart which illustrates in detail substeps of block 510 of the preferred method of FIG. 11;

- FIGs. 14 schematically illustrates a marker 40 according to a preferred embodiment of the invention, with geometric features highlighted;

- FIG. 15 illustrates one of the geometrical features highlighted in FIG. 14; and

- FIGs. 16 and 17 illustrate relative reference systems defined on glyphs of a marker, used in steps of a method according to a preferred embodiment of the invention.

Detailed description of currently preferred embodiments

Throughout the various embodiments disclosed herein, similar elements or elements having a similar function are indicated by the same reference numbers.

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures associated with the various embodiments have not been shown or described in detail to avoid unnecessarily obscuring descriptions of the embodiments.

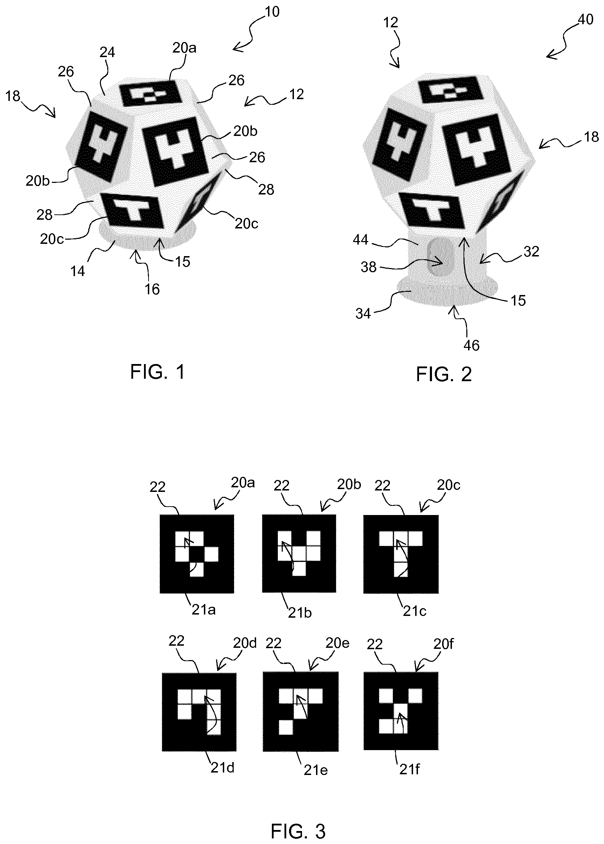

With reference to FIGs. 1-5, preferred embodiments of three-dimensional markers 10, 40 according to the invention, used to carry out a related preferred method later outlined, are now described.

The marker 10 illustrated in FIG. 1 comprises a main body 12 shaped as a regular polyhedron, and a base 14.

The main body 12 is preferably shaped as a dodecahedron, as illustrated, and thus comprises twelve faces shaped as regular pentagons. In alternative embodiments not shown, the main body 12 can be shaped as a different regular polyhedron, such as for example an octahedron, an icosahedron, etc.

The base 14 comprises a contact surface 16 configured to contact and be attached to the skin of a human or animal subject at specific locations, corresponding to predefined body landmarks of an anatomical structure of the skeleton of the subject of interest.

In the case illustrated, the base 14 is in particular a disc-shaped plate stably connected to a proximal face 15 of the main body 12, and the contact surface 16 is thus substantially circular.

The base 14 can be integral with the main body 12 of the marker 10, or it can be a distinct element stably coupled therewith, releasably or unreleasably, by providing suitable connecting elements (not visible) at the interface with the main body 12 of the marker 10. By way of example, the base 14 can be coupled to the main body 12 by means of gluing, interference fit or other types of coupling.

Markers 10 are preferably attached to the skin of the subject, through the base 14, by means of a suitable hypoallergenic adhesive (not shown). By way of a non limiting example, a double sided adhesive sheet made of a polymeric material can be employed. Such adhesive sheet can be advantageously pre-attached to the contact surface 16, equipped with a release sheet to be peeled off before applying the marker 10 onto the subject.

An example of a suitable adhesive sheet is a 20mm circular adhesive pad provided by 3M.

In the present disclosure, the other faces of the main body 12, except the proximal face 15 connected to the base 14, are all referred to as exposed faces 18.

Each exposed face 18 has a respective distinctive feature 20a, 20b, 20c provided thereon. Distinctive features 20a, 20b, 20c are in particular glyphs (referred to using the same reference numbers 20a, 20b, 20c), namely simple graphic elements that are recognizable by processing the image according to a suitable glyph recognition algorithm. Such glyphs can be for example printed on the faces of the main body of the markers.

In the preferred embodiments illustrated, a distal face 24, opposite to the proximal face 15 of the polyhedral main body 12 of the marker 10, has a first glyph referred to as distal glyph 20a; the five faces immediately adjacent to the distal face 24, referred to as first lateral faces 26, have a second common glyph, referred to as first lateral glyph 20b; and the five faces immediately adjacent to the base 14, referred to as second lateral faces 28, have a third common glyph, referred to as second lateral glyph 20c.

Glyphs 20a, 20b, 20c, better illustrated in FIG. 3 along with other exemplary glyphs 20d, 20e, 20f according to preferred embodiments of the invention, each comprise a graphic sign 21a-21f over a background 22 having a contrasting colour. Signs 21a-21f are in particular of light color over a dark background 22, so that the overall glyphs 20a, 20b, 20c are emphasized with respect to the outer surface of the main body 12 of the marker 10, which is also light in color (see FIG. 1). In different embodiments (not shown), inverted colors can of course be provided.

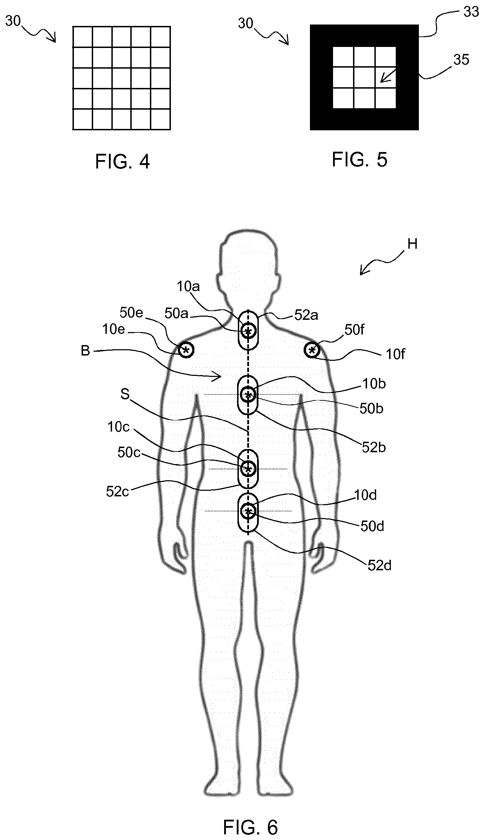

Glyphs 20a-20f are obtained by suitably distributing dark squared modules within a grid (e.g. grid 30 shown in FIGs. 4-5), by first filling the peripheral rows and columns with dark modules 33 (FIG. 5), and by further adding other dark modules in the remaining space 35 in the center until a desired glyph is obtained, with the provision that for each row and column within space 35, at least one block of the grid 30 is left unfilled.

By way of example, the grid 30 shown in FIGs. 4-5 is a 5x5 grid, but grids of other dimensions, such as 6x6 grids, could be used instead.

The provision of a marker 10 having a three-dimensional main body 12, projecting at a certain distance from the skin of the subject when the marker is applied thereon, allows the marker 10 to be at least partially visible also when it is applied in body regions of the subject that might be subject to visual obstruction - when viewed from certain angles - due to the presence of protruding body parts, such as the scapulae.

FIG. 2 shows a marker 40 according to a second preferred embodiment of the invention. The marker 40 differs from marker 10 only in that the base 44 comprises an elongated stem 32 projecting from the proximal face 15 of the main body 12 of the marker 40, and terminating with a disc-shaped plate 34 on which the contact surface 46 with the skin of the subject, circular in shape, is defined.

The stem 32 is preferably shaped as a hollow cylinder, and can comprise, as illustrated, one or more openings 38 that lighten the overall marker 40. The length of the stem 32 can be tailored based on a desired projecting distance of the marker 40 with respect to the skin of the subject, so that the marker 40 may be suitable for use also in regions of the body of the subject that are particularly subject to visual obstruction.

It should be noted that although markers 10, 40 have been described as comprising a distinct base 14, 44 on which the contact surface 16, 46 of the markers with the skin of the subject is defined, in simpler embodiments (not shown) the contact surface can be embodied by the proximal face 15 itself of the main body 12.

The markers 10, 40 can be manufactured from any hypoallergenic light, rigid material, and both the main body 12 and the stem 32, where provided, can be solid or hollow. Light plastic materials are particularly suitable for manufacturing the markers.

Preferably, the markers 10, 40 are made of a polymeric material.

More preferably, the polymeric material is selected from poly-lactic acid (PLA), acrylonitrile-butadiene-styrene (ABS), high impact polystyrene (HIPS), nylon, carbon fiber, polycarbonate and acrylic styrene acrylonitrile (ASA).

Preferably, in such a case the markers 10, 40 can advantageously be manufactured using 3D printing processes.

Different marker sizes, along with glyphs dimensions, can be provided.

By way of example, the main body of the markers 10, 40 may have an edge of about 12 mm and can be inscribed in a sphere having a diameter of about 33 mm.

By way of example, the contact surface 16, 46 of the markers 10, 40 may be circular and may have a diameter of about 21 mm.

By way of example, a distance between the proximal face 15 and the contact surface 16 of the marker 10 is of about 6 mm.

By way of example, a distance between the proximal face 15 and the contact surface 46 of the marker 40 is of about 20 mm.

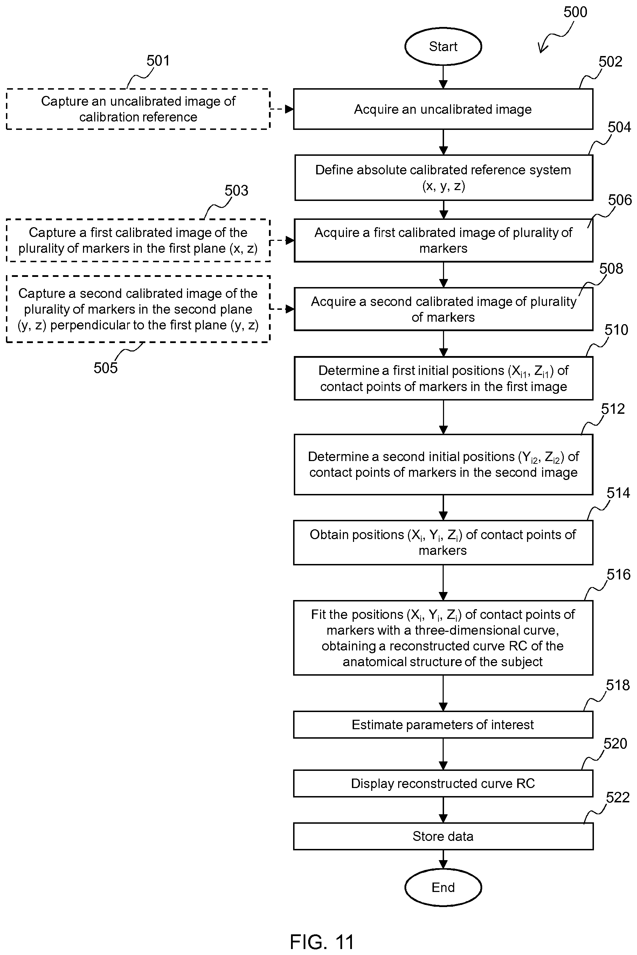

The markers 10, 40 are employed in the method of the invention, as later described in detail with reference to FIGs. 11-17, to determine a pattern of points of a wide variety of different anatomical structures of human or animal subjects, and to subsequently extract biometric data from such pattern of points. To this end, a plurality of markers 10, 40 is distributed on the subject at specific body landmarks.

The number of markers 10, 40 employed and the selection of body landmarks depends on the complexity of the anatomical structure under analysis and on the desired biometric parameters to be subsequently estimated.

For example, for dimensional analysis of simple "linear" structures such as long bones (e.g. femur, tibia), two markers, positioned at the bone ends, are sufficient. For example, for leg length analysis, relevant landmarks are the trochanter and the outer malleolus.

For more complex structures, such as the spine, more than two markers are generally needed.

FIG. 6 schematically illustrates an exemplary distribution of markers on the skin of a human subject H for biometric analysis of the spine, according to a preferred embodiment of the invention.

The markers may be markers according to any embodiment of markers 10, 40 described above with reference to FIGs. 1-5, preferably having a main body 12 of a same size. The markers may be of a same type or of a different type.

In FIG. 6, markers 10a, 10b, 10c and 10d are shown merely as an example.

In a different embodiment, the markers 40 comprising the stem 32 (see FIG. 2) may be employed on some or all the body landmarks, especially in those regions of the body that are particularly subject to visual obstruction when observed "laterally".

As shown in FIG. 6, four markers 10a, 10b, 10c and 10d are applied on the back B of the subject H at a corresponding plurality of body landmarks 50a, 50b, 50c, 50d located along the spine S.

In particular, a first body landmark 50a is selected among cervical vertebrae C4 and C5 (approximately within region 52a); a second body landmark 50b is selected among dorsal vertebrae D4, D5, D6 and D7 (approximately within region 52b); a third body landmark 50c is selected among dorsal vertebrae D8, D9, D10, D11 and D12 (approximately within region 52c); and a fourth body landmark 50d is selected among lumbar vertebrae L1, L2, L3, L4 and L5 (approximately within region 52d).

Additional markers 10e, 10f can be optionally provided to improve the analysis by correlating the morphology and position of the spine S with the surrounding bone structures of the torso. For example, as illustrated, additional markers 10e, 10f are provided respectively positioned at body landmarks 50e, 50f, corresponding to the left and right acromions.

In an even more complex scenario, for analyzing the overall skeleton, ("total body" analysis), up to twenty markers may be needed, according to another embodiment (not shown) of the invention.

Besides the six markers 10a-10f illustrated in FIG. 6, positioned along the spine and at the acromions, further markers (not shown) may be added, the related body landmarks being preferably selected from: apex of left and right axillary cavities, lower apex of left and right scapulae, left and right posterior superior iliac spines, left and right olecranons, left and right lateral knee, left and right outer malleolus, left and right anterior superior iliac spines, left and right radial styloid processes, left and right medial malleolus.

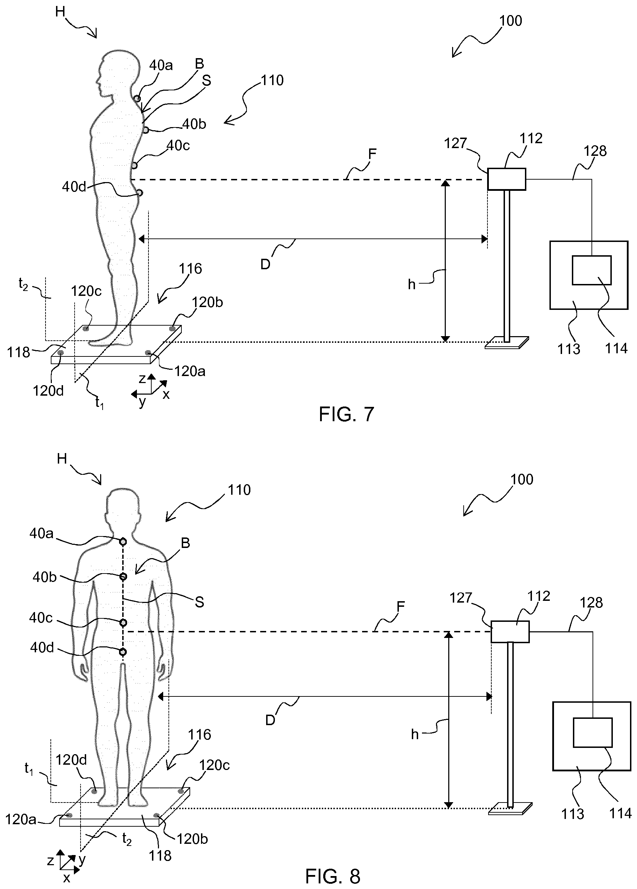

FIGs. 7-8 schematically illustrate a system 100 according to a preferred embodiment of the invention, configured to carry out a preferred method, later described, for obtaining a spatial pattern of an anatomical structure of a subject H, in particular a human subject H.

The system 100 preferably comprises a plurality 110 of markers (which may be the markers 10, the markers 40 or any suitable combination thereof), a digital image capturing device 112, in particular a camera, a computational device 113 including a processor 114, and a support 116.

In the preferred embodiment illustrated in FIGs. 7-8 and, once again, merely as an example, the plurality 110 of markers includes the markers 40 described above.

In particular, the plurality 110 of markers illustrated includes the previously described markers 40a-40d, applied on the back B of the subject H and distributed along the spine S, in a same manner as previously illustrated in FIG. 6.

The system 100 illustrated in FIGs. 7-8 is thus set for analyzing the spine S of the subject H, according to a preferred embodiment of the method of the invention.

Preferably, the support 116 is adapted to receive the subject H, having the markers 40a-40d attached on the spine at selected body landmarks, in a standardized standing position. The camera 112 is configured and positioned to capture images of the plurality 110 of markers, applied onto the subject H, from at least two different angles.

Images captured by the camera 112 are transferred to the processor 114 of the computational device 113 for image processing and subsequent elaboration in order to reconstruct a pattern of points of the spine S of the subject H and further determine related biometric parameters.

Preferably, the support 116 is rotatably mounted onto a stationary base (underneath the support 116 and not visible) that lays on the ground, so that the support 116 is rotatable with respect to the stationary base.

Preferably, the support 116 is rotatable between fixed angular positions angularly spaced at 90° from each other.

Preferably, the support 116 is a squared platform with a substantially flat upper surface 118.

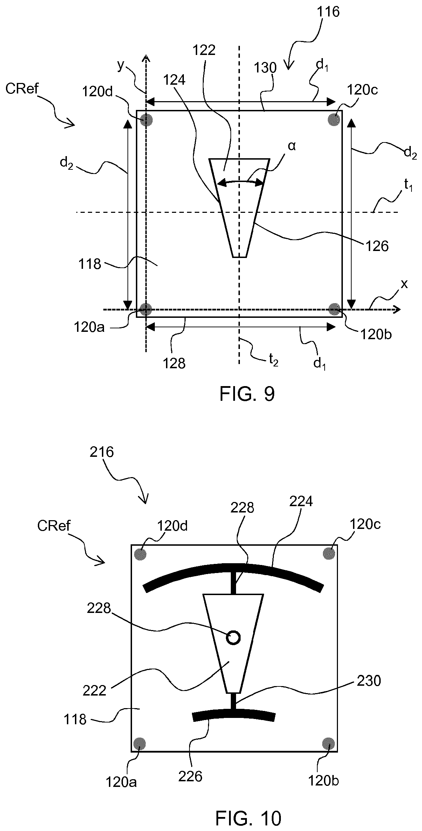

With further reference to FIG. 9, it may be appreciated that the support 116 preferably comprises, on the upper surface 118 thereof, four tags 120a, 120b, 120c, and 120d of a reflective material.

Conveniently, the tags 120a, 120b, 120c, 120d constitute a calibration reference CRef used during a calibration operation of the method of the invention, as later described.

The tags 120a, 120b, 120c, 120d of the calibration reference CRef are positioned at the four corners of the upper surface 118 of the support 116. Therefore, each tag is equally spaced from adjacent ones of a distance $d_1 = d_2$, and couples of tags 120a, 120b; 120b, 120c; 120c, 120d; and 120d, 120a lie along reciprocally perpendicular directions (axes x and y), respectively corresponding to a latero-lateral direction and to an postero-anterior direction of the subject H standing on the support 116. Moreover, the perpendicular directions x, y are respectively parallel to median planes $t_1$ and $t_2$ of the support 116.

Preferably, the support 116 further comprises, fixed on its upper surface 118, a guide 122 (illustrated only in FIG. 9 for the sake of clarity) comprising angled or oblique sides 124, 126 that diverge from one another moving from a posterior side 128 to an anterior side 130 of the support 116.

The oblique sides 124, 126 of the guide 122 are configured to abut, in use, to inner sides of the feet of the subject (not shown) in order to fix and "standardize" the position of the subject. The angle $\alpha$ defined by the sides 124, 126 is of about 30°, which is considered an optimum angle to evaluate postural issues.

In a different preferred embodiment shown in FIG. 10, a support 216 comprises a guide 222 that incorporates a length limiter formed by anterior and posterior bars 224, 226 connected to the guide 222 by means of connecting shanks 228, 230. Bars 224, 226 can be approached or moved away from each other by acting on a displacing mechanism driven by a suitable controller such as a dial 228, correspondingly reducing or increasing a distance therebetween in order to adapt the bars 224, 226 to different feet sizes.

Going back to FIGs. 7-8, the camera 112 is configured to capture images of the plurality 110 of markers 40a-40d applied to the skin of subject H.

The camera 112 is positioned so that its focal axis F is substantially parallel to the upper surface 118 of the support 116, namely so that an angle between the focal axis F and the upper surface 118 of the support 116 is comprised between 0° and 1°.

The focal axis F is also substantially aligned with either median plane $t_2$ (image capturing configuration of FIG. 7) or median plane $t_1$ (image capturing configuration of FIG. 8), respectively, with a maximum lateral displacement with respect to median plane $t_2$ or median plane $t_1$, respectively, comprised within the range of $\pm$ 5 mm.

Preferably, the camera 112 is so positioned that its lens 127 is at a predefined distance D with respect to median plane $t_1$ (configuration of FIG. 7), respectively median plane $t_2$ (configuration of FIG. 8) of the support 116. The distance D is preferably comprised between 235 and 290 cm.

Moreover, based on the height of the subject H, the camera 112 is positioned so that the focal axis F is at a vertical distance h from the upper surface of the support 116, said vertical distance h preferably substantially corresponding to the height of the subject's iliac crest.

In this way, the image distortion is advantageously minimized.

In the image capturing configuration of FIG. 7, the camera 112 captures a first calibrated image depicting a spatial arrangement of the markers 40a-40d in a first plane (x, z) substantially parallel to (or coinciding with) the median plane $t_1$ of the support 116.

Preferably, this first plane (x, z) corresponds to a coronal plane of the subject H carrying the markers 40a-40d.

In other words, in the image capturing configuration of FIG. 7, the camera 112 captures the back B of the subject H, carrying the markers 40a-40d right from behind, along a direction substantially coinciding with a postero-anterior direction of the subject H.

In the image capturing configuration of FIG. 8, the camera 112 captures a second calibrated image depicting a spatial arrangement of the markers 40a-40d in a second plane (y, z) substantially parallel to (or coinciding with) the median plane $t_2$ and perpendicular to the median plane $t_1$ of the support 116.

Preferably, this second plane (y, z) corresponds to a sagittal plane of the subject H carrying the markers 40a-40d.

In other words, in the image capturing configuration of FIG. 8, the camera 112 captures "laterally" the back B of the subject H, carrying the markers 40a-40d, along a direction substantially coinciding with a latero-lateral direction of the subject H.

By way of example, the camera is a Canon 5Ds with 50 megapixel sensor and a 18-55 lens.

The images captured by the camera 112 are then acquired by the processor 114 of the computational device 113, in order to be processed according to a preferred method according to the invention.

The processor 114 can be in data communication with the camera 112 through a suitable communication link 128, such as a cable or a wireless link.

Alternatively, data transfer between the camera 112 and the processor 114 can take place off-line, by means of movable storage devices such as USB flash drives or flash memory cards to be placed in suitable ports/readers provided in the computational device 113 and in the camera 112.

The processor 114, which is for example a central processor (CPU) of the computational device 113, may be coupled to a random access memory (RAM), preferably of at least 8GB, and to a read-only memory (ROM). The ROM may also be other types of storage media to store programs, such as programmable ROM (PROM), erasable PROM (EPROM), etc.

The computational device 113 may include a hard drive, preferably of at least 256 GB.

The processor 114 may communicate with other internal and/or external components through input/output (I/O) circuitry and bussing to provide control signals and the like. The processor 114 carries out a variety of functions as is known in the art, as dictated by software and/or firmware instructions.

The computational device 113 may also include one or more data storage devices, such as a hard disk drive, flash memory drive, CD-ROM drive and/or other hardware capable of reading and/or storing information.

Software may be stored in the read-only memory of the computational device 113 and/or distributed on a removable memory device or other form of media capable of portably storing information. These storage media may be inserted into, and read by, devices such as the CD-ROM drive, the disk drive, USB drive etc.

The processor 114 may be coupled to a display, which may be any type of known display or presentation screen, such as LCD displays, LED displays, plasma display, cathode ray tubes (CRT), etc. A user input interface may be provided, including one or more user interface mechanisms such as a mouse, keyboard, microphone, touch pad, touch screen, voice-recognition system, etc.

The processor 114 may be coupled to other computing devices, such as the landline and/or wireless terminals via a network.

The computational device 113 may be part of a larger network configuration as in a global area network (GAN) such as the Internet, which allows ultimate connection to the various landline and/or mobile client devices.

For example, the processor 114 of the computational device 113 may communicate with one or more remote processors.

The computational device 113 can be for example a personal computer (PC), a tablet or even a sufficiently performing smartphone.

In case of a PC, preferably the computer runs with a Windows operating system, more preferably with Windows 10Pro or upper versions, and the hard disk drive is preferably of at least 256 GB.

With reference to FIGs. 11-17, a method 500 for obtaining a spatial pattern of an anatomical structure of a subject according to a preferred embodiment of the invention is now described.

The method 500 is preferably carried out by the system 100 of FIGs. 7-8. In the description below, reference will be made sometimes to the system 100 and related components, indicated by the same reference numbers as used above in the related description.

As already stated, the method 500 of the invention allows to easily acquire photographic images of a subject H having a plurality 110 of markers attached to the skin thereof at predefined anatomical landmarks of an anatomical structure of interest, and thereby allows to easily determine a "pattern of points" of such anatomical structure, based on which parameters of interest may be extracted.

In FIG. 11, even blocks 502-522, bound by solid lines, represent the main steps of the method 500, executed by the processor 114. Odd blocks 501-505, bound by dashed lines, are further operations executed by the camera 112, added to better support the description of the method 500.

In block 502, an uncalibrated image depicting a calibration reference is acquired. The uncalibrated image depicts in particular the support 116 carrying on its upper surface 118 the calibration reference CRef, constituted by the tags 120a-120d (shown in FIGs. 7-10).

The uncalibrated image is captured (block 501) by the camera 112 positioned with respect to the support 116 in a similar image capturing configuration as that of FIG. 7, but without the subject H standing over the support 116.

Based on the uncalibrated image acquired in block 502, in block 504 an image calibration operation is carried out, through which an absolute calibrated reference system (x, y, z) is defined.

In the calibration operation of block 504, axes x, y are first defined based on the aligned tags 120a-120d (see in particular FIG. 9). In particular, axis x is set as the direction along which tags 120a and 120b lay, and axis y is set as the direction along which tags 120a and 120d lay. The direction of the third axis z is then derived as the vertical axis perpendicular to the other two axes x, y.

Moreover, a [pixel] to [cm] conversion factor is determined based on the known resolution of the camera 112, and on the known distance $d_1$, $d_2$ between the tags 120a-120b; 120a-120d positioned on the support 116.

In step 506, a first calibrated image of a plurality 110 of markers 40a-40d applied over the skin of the subject H at the relevant body landmarks of the spine S is acquired.

The first calibrated image is captured (block 503) by the camera 112 positioned with respect to the subject H as in the image capturing configuration illustrated in FIG. 7, in which the back B of the subject H is captured right from behind.

As stated above, the first calibrated image depicts a marker spatial arrangement in the first plane (x, z) substantially parallel to (or coinciding with) the median plane $t_1$ of the support 116.

In order to obtain a good three-dimensional reconstruction of the spine, from which parameters of interest may be reliably estimated, each of the markers 40a-40d should be attached at the relevant body landmark precisely enough so that the contact point of the marker - substantially corresponding to the center of the contact surface 46 of the marker - matches as closely as possible the body landmark on which the marker is to be applied.

To this end, positioning of the markers 40a-40d is preferably carried out by qualified staff, or at least by specifically trained staff.

In block 508, a second calibrated image of the plurality 110 of markers applied over the skin of the subject H at the relevant body landmarks of the spine S is acquired.

The second calibrated image is captured (block 505) by the camera 112 in the image capturing configuration illustrated in FIG. 8, in which the back B of the subject H is captured "laterally".

As stated above, the second calibrated image depicts a marker spatial arrangement in the second plane (y, z) substantially parallel to median plane $t_2$ and perpendicular to median plane $t_1$.

In a preferred embodiment, to pass from the image capturing configuration of FIG. 7 to that of FIG. 8, the subject H is rotated 90° clockwise about a vertical axis, for example by exploiting the rotating mechanism provided in the support 116, 216 on which he stands.

In this way, the camera 112 remains advantageously stationary and no adjustments nor resettings are necessary.

A person skilled in the art will understand that instead of rotating the subject H, it is equally possible to move the camera 112 with respect thereto.

In subsequent blocks 510 and 512, first and second initial positions of contact points of each marker 40a-40d with the respective body landmark 50a-50d of the subject H are determined respectively in the first and second calibrated images, acquired in blocks 506, 508 and each captured in a respective one of the perpendicular first and second planes (x, z; y, z).

After execution of blocks 510, 512, two sets of two-dimensional coordinates $(X_{i-1}, Z_{i-1})$ and $(Y_{i-2}, Z_{i-2})$ in the absolute coordinate reference system, related to the first and second initial positions of contact points of each marker, are obtained.

Coordinates $(X_{i-1}, Z_{i-1})$ and $(Y_{i-2}, Z_{i-2})$, in which index "i" is an integer spanning between 1 and the total number of markers (4 in the case illustrated in FIGs. 7-8) are also referred to as 2D coordinates.

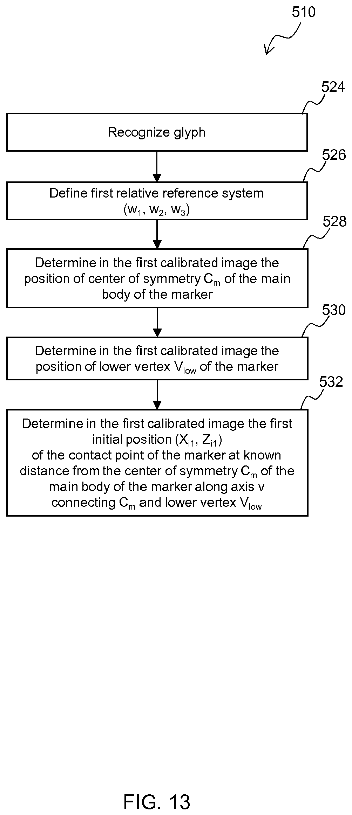

The detailed operations carried out in blocks 510 and 512 are outlined hereinafter in connection with FIGs. 13-17.

In block 514, positions $(X_i, Y_i, Z_i)$ of contact points of each marker 40a-40d, expressed by three-dimensional coordinates in the absolute calibrated reference system (x, y, z) and also referred to as 3D coordinates, in contrast with the first and second 2D coordinates, are determined by combining, for each marker, the related first and second initial positions $(X_{i-1}, Z_{i-1})$ and $(Y_{i-2}, Z_{i-2})$ of contact points, according to the following formulas (1) to (3).

$$X_i = X_{i-1} \qquad\qquad\qquad\qquad\qquad (1)$$

$$Y_i = Y_{i-2} \qquad\qquad\qquad\qquad\qquad (2)$$

$$Z_i = (Z_{i-1} + Z_{i-2}) / 2 \qquad\qquad\qquad\qquad (3)$$

In detail, the 3D coordinate $Z_i$ is determined by averaging the common first and second 2D coordinates $Z_{i-1}$ and $Z_{i-2}$, whereas the other two 3D coordinates $X_i$ and $Y_i$ are set as the corresponding 2D coordinates $X_{i-1}$ and $Y_{i-2}$ respectively determined from the first and second calibrated images.

In subsequent block 516, the positions $(X_i, Y_i, Z_i)$ of contact points of all markers 40a-40d are fitted with a three-dimensional curve, which constitutes a three-dimensional pattern of the spine S of the subject H.

The fitting operation of block 516 is carried out by means of a suitable interpolation algorithm such as polynomial interpolation or spline interpolation. Preferably, cubic spline interpolations is used.

As known, given a set of n+1 different data points or nodes falling within a main numerical interval and dividing the main numerical interval in n sub-intervals, the cubic spline s(x) is the function defined piecewise by n third-order polynomials, each defined in each of the n sub-intervals.

The second derivative of each polynomial is set to zero at the endpoints of each of the n sub-intervals, to ensure a smooth curvature of the spline s(x) at the "junction" between each couple of polynomials.

In the case analyzed, the positions $(X_i, Y_i, Z_i)$ of contact points of the four markers 40a-40d are set as nodes, the first and the last position along the spine representing the ends of the main numerical interval.

Further data points may be added, within the main interval, so as to define narrower sub-intervals and thereby obtain a smoother curve.

By way of example, the spline s(x) may be defined by 999 polynomials defined in 999 corresponding sub-intervals delimited by 1000 points. Further data points besides the four positions of the markers may be found by setting up a matrix system.

The fitting operation of block 516 yields a three-dimensional curve that represents a pattern of the spine S of subject H, that will be referred to hereinafter as reconstructed curve or RC.

In subsequent block 518, parameters of interest are estimated from the reconstructed curve determined in block 516.

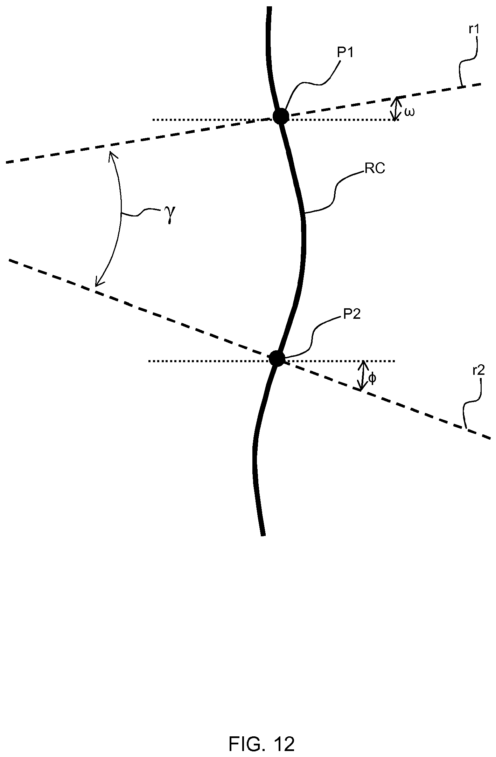

A parameter of particular interest that can be extracted from the reconstructed curve of the spine is the Cobb angle $\gamma$, as schematically illustrated in FIG. 12.

As known in the art, the Cobb angle is defined as the greatest angle at a certain region of the vertebral column, conventionally measured in X-ray images of the spine in the coronal plane, measured from the superior endplate of a predetermine superior vertebra to the inferior endplate of a predetermined inferior vertebra. The Cobb angle allows to estimate the entity of bending disorders of the spine (e.g. scoliosis), both due to postural issues and to traumatic events.

The reconstructed curve RC in FIG. 12 is viewed in the coronal plane, therefore it can be represented, in this case, as a one-variable function f(x).

First, to determine the Cobb angle of the reconstructed curve RC, changes of concavity along the reconstructed curve are preferably determined by finding the zero-derivative points (e.g. P1 and P2 in FIG. 12) of the function f(x), calculated by imposing a null second derivative of the function f(x), according to formula (4):

$$f'' = d^2f / dx^2 = 0 \qquad\qquad (4)$$

Thereafter, the equations of straight lines $r_1$ and $r_2$ orthogonal to the reconstructed curve RC at the zero-derivative points P1 and P2 are determined according to the following formulas (5) and (6) (in which the y-intercept is neglected for simplicity):

$$r_1 = m_1x \qquad\qquad (5)$$

$$r_2 = m_2x \qquad\qquad (6)$$

with

$$m_1 = \tan(\omega), m_2 = \tan(\phi)$$

The values of angles $\omega$ and $\phi$ are determined based on the first derivative of the zero-derivative points P1 and P2.

Cobb angle $\gamma$ is thus determined according to the following formula (7):

$$\gamma = \arctan|(m_1-m_2)/(1+m_1m_2)| \qquad\qquad (7)$$

According to the knowledge in the art, scoliosis is generally associated to a Cobb angle of more than 10°.

Going back to FIG. 11, after the parameter extraction of block 518, in block 520 the reconstructed curve RC can be further displayed, for example on a display of the computational device 113.

In particular, the reconstructed curve RC may be displayed as it is (in the form of a simple three-dimensional "line") to appreciate a pattern of the spine S, or in more advanced applications it might be used to create a proper three-dimensional model of the spine S, including 3D-modeled bone structures.

Such three-dimensional model may be built, based on standard three-dimensional templates of the human spine (and possibly of neighbouring bone structures, e.g. the pelvis), by setting the reciprocal spatial arrangement of the various bones according to the pattern defined by the reconstructed curve RC.

The three-dimensional model can be built according to any suitable 3D modeling algorithm.

Examples of commercial 3D-modeling software that are nowadays available and could suitably be used to build a similar model of the spine are, i.e., GameStudio, Autodesk Maya, Autodesk Mudbox, Houdini, Cinema 4D, Modo, Autodesk 3Ds Max, ZBrush, Rhinoceros, Lightwave 3D, 3DCoat, Blender, Daz Studio Hexagon, Fusion 360, Houdini Apprentice, Wings3D, Rocket 3F, Sculptris.

Finally, in step 522 data related to the reconstructed curve RC, as well as the parameters extracted in block 518, are preferably stored in the memory of the computational device 113 and/or in a remote central database.

With reference to FIGs. 13-17, the step of determining the first initial positions of block 510 is described in detail.

The following steps are carried out for each i-th marker 40a-40d depicted in the first calibrated image, acquired in block 506.

Block 510 comprises a sub-block 524 in which, for each marker 40a-40d, one or more glyphs 20a-20f (see FIG. 3) on the outer surface thereof are recognized by running a predetermined glyph recognition algorithm.

The memory of the computational device 113 stores a database in which each glyph is associated with the face or group of faces on which it is applied/printed, along with information related to the geometry of the marker 40a-40d. For each marker 40a-40d in the first calibrated image, the recognition algorithm detects and recognizes one or more glyphs and associates the glyph to the face on which it is provided.

The predetermined glyph recognition algorithm can be implemented by any suitable commercial or non-commercial software for optical glyph recognition, preferably comprising the steps of:

- grayscaling the first calibrated image;

- detecting one or more glyphs by recognizing their quadrilateral dark shape emerging against the respective white faces of the marker on which they are applied;

- "binarizing" each detected glyph by dividing it into a grid of cells having same width and height (e.g. grid 30 of FIG. 3) and assigning a value "0" or "1" to each cell of the grid based on a prevalence of white or dark/black pixels within the cell, thereby expressing the detected glyph as a binary matrix;

- matching the detected glyph with the database of glyphs, thereby recognizing the glyph and its associated face of the marker.

Based on the number of glyphs recognized in the first calibrated image through the glyph recognition algorithm (block 524), in following blocks 526-532 the contact point of each of the markers 40a-40d is preferably determined through different geometrical calculations, according to the known geometry of the regular dodecahedral main body of the marker 40a-40d.

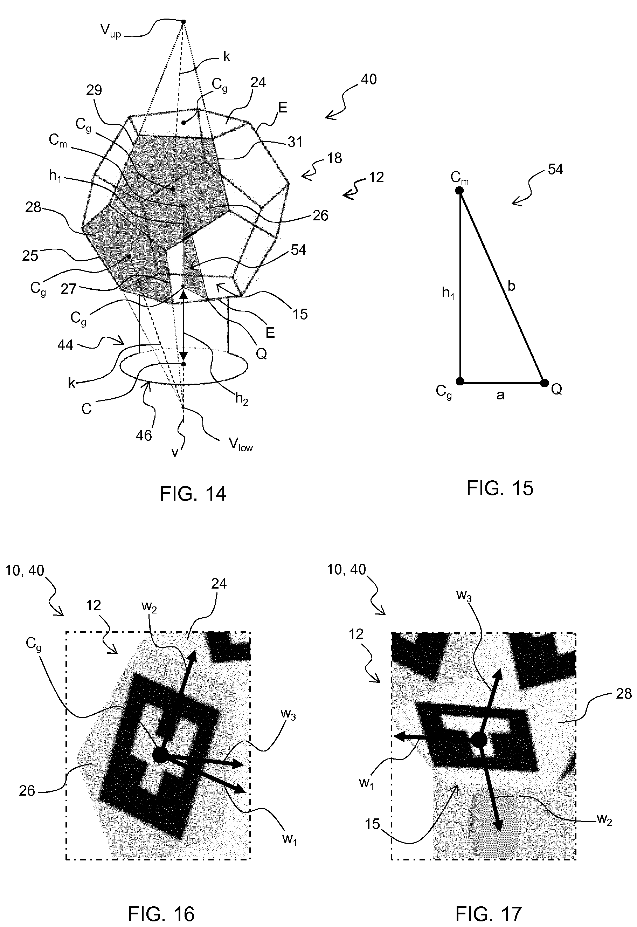

Relevant geometric features of the dodecahedral main body 12 of a marker 40 (see also FIG. 2) according to the invention are highlighted in FIG. 14. Glyphs on the exposed faces 18 of the marker 40 are not shown in this figure for the sake of clarity.

Based on the known length of the edge E of the regular dodecahedral main body 12 and on the known relevant angles thereof, the dimensions of the sides a, $h_1$, b of the right-angled triangle 54 (cf. also FIG. 15) having as vertexes the centre of symmetry of the marker $C_m$, the centre of symmetry $C_g$ of the proximal face 15 (corresponding to the center of symmetry of the glyph thereon, not shown) and the medium point Q of an edge E of the face 15, are found by trigonometric calculations, according to the following formulas (8)-(10):

$$a = 0{,}85 \times E \tag{8}$$

$$h_1 = 1{,}11 \times E \tag{9}$$

$$b = 1{,}40 \times E \tag{10}$$

The same applies for analogous triangles construed on each face and on each edge of the dodecahedral main body 12 of the marker 40.

Still according to trigonometric calculations, the distance k between the centre of symmetry $C_g$ of the second lateral face 28 and the "lower vertex" $V_{low}$, namely the intersection point between proximal extensions of oblique sides 25, 27 of a second lateral face 28, is determined according to formula (11):

$$k = 2{,}23 \times E \tag{11}$$

The same distance k is defined between the centre of symmetry $C_g$ of the first lateral face 26 and the "upper vertex" $V_{up}$, namely the intersection point between distal extensions of oblique sides 29, 31 of a first lateral face 26.

These geometric features are exploited in the steps of the method 500 outlined below, for determining the contact point C of the marker 40a-40d with the respective body landmark.

Going back to FIG. 13, in block 526 a first relative reference system ($w_1$, $w_2$, $w_3$) is defined (cf. FIGs. 16-17), preferably having its origin centered at the center of symmetry $C_g$ of each glyph recognized.

Preferably, the axes $w_1$ and $w_2$ are oriented parallel to sides of the glyph, whereas the axis $w_3$ is oriented towards inside the marker.

In particular, for a first lateral glyph provided on a first lateral face 26 (FIG. 16), axis $w_2$ is preferably oriented towards the distal face 24 of the main body 12, whereas for a second lateral glyph provided on a second lateral face 28 (FIG. 17), axis $w_2$ is preferably oriented towards the proximal face 15 of the main body 12 of the marker.

In block 528, the position of center of symmetry $C_m$ of the main body of each marker 110 is determined in the first calibrated image.

Block 528 preferably involves different operations based on the glyph(s) recognized, as detailed below.

If only the distal glyph provided on the distal face 24 is recognized, the center of symmetry $C_m$ of the main body of the marker is determined, in the first calibrated image, at coordinates (0, 0, 0) in the first relative reference system ($w_1$, $w_2$, $w_3$), namely it is approximated to correspond to the centre of symmetry $C_g$ of the distal glyph on distal face 24 recognized.

If only one of the first or second lateral glyphs, provided either on a first lateral face 26 or on a second lateral face 28, is recognized in the first calibrated image, the center of symmetry $C_m$ of the main body of the marker is determined at

coordinates $(0, 0, h_1)$ in the first relative reference system $(w_1, w_2, w_3)$.

If two glyphs are recognized in the first calibrated image, the center of symmetry $C_m$ of the main body of the marker is determined at the intersection point of the axes $w_3$ of the first relative reference systems $(w_1, w_2, w_3)$ defined for each glyph.

If three glyphs are recognized in the first calibrated image, relative coordinates of the center of symmetry $C_m$ of the main body 12 of the marker are determined by averaging the coordinates of the intersection points between the axes $w_3$ of the first relative reference systems $(w_1, w_2, w_3)$ of all the possible pairs of glyphs.

If four or more glyphs are recognized in the first calibrated image, relative coordinates of the center of symmetry $C_m$ of the main body 12 of the marker are determined by weight averaging the coordinates of the intersection points between the axes $w_3$ of the first relative reference systems $(w_1, w_2, w_3)$ of all the possible pairs of glyphs.

In subsequent block 530, the position of the lower vertex $V_{low}$ of the marker, as described above with reference to FIG. 14, is determined in the first calibrated image.

Block 530 involves different operations based on the glyph(s) recognized in the first calibrated image, as detailed below.

If only the distal glyph provided on the distal face 24 is recognized, the lower vertex $V_{low}$ of the main body of the marker is determined, in the first calibrated image, at coordinates $(0, 0, 0)$ in the first relative reference system $(w_1, w_2, w_3)$, namely it is approximated to correspond to the centre of symmetry $C_g$ of the glyph.

If only one first lateral glyph provided on a first lateral face 26 is recognized in the first calibrated image, the upper vertex $V_{up}$ is determined at coordinates $(0, k, 0)$ in the first relative reference system $(w_1, w_2, w_3)$. The lower vertex $V_{low}$ is then determined as the symmetrical point of said upper vertex $V_{up}$ with respect to the centre of symmetry $C_m$ of the main body of the marker.

If only one second lateral glyph provided on a second lateral face 28 is recognized in the first calibrated image, the lower vertex $V_{low}$ is determined at coordinates $(0, k, 0)$ in the first relative reference system $(w_1, w_2, w_3)$.

If two first lateral glyphs provided on a first lateral face 26 are recognized in the first calibrated image, the upper vertex $V_{up}$ of the marker is determined at the intersection point of the axes $w_2$ of the first relative reference systems $(w_1, w_2, w_3)$ defined for each of the two first lateral glyphs. The lower vertex $V_{low}$ is then determined as the symmetrical point of said upper vertex $V_{up}$ with respect to the centre of symmetry $C_m$ of the main body 12 of the marker.

If two second lateral glyphs provided on a second lateral face 28 are recognized in the first calibrated image, the lower vertex $V_{low}$ of the marker is determined at the intersection point of the axes $w_2$ of the first relative reference systems $(w_1, w_2, w_3)$ defined for each of the two second lateral glyphs.

If the distal glyph, provided on the distal face 24, and one first lateral glyph, provided on a first lateral face 26, are recognized in the first calibrated image, the upper vertex $V_{up}$ is determined at the intersection point between axis $w_3$ of the first relative reference system $(w_1, w_2, w_3)$ of the distal glyph and axis $w_2$ of the first relative reference system $(w_1, w_2, w_3)$ of the first lateral glyph. The lower vertex $V_{low}$ is then determined as the symmetrical point of said upper vertex $V_{up}$ with respect to the centre of symmetry $C_m$ of the main body 12 of the marker.

If the distal glyph, provided on the distal face 24, and one second lateral glyph, provided on a second lateral face 28, are recognized in the first calibrated image, the lower vertex $V_{low}$ is determined at the intersection point between axis $w_3$ of the distal glyph and axis $w_2$ of the first relative reference system $(w_1, w_2, w_3)$ of the first lateral glyph.

All other cases are preferably treated as combinations of two or more of the cases above, and the "final" lower vertex $V_{low}$ is determined by weight averaging the position of $V_{low}$ obtained in each case above.

In the averaging or weight averaging operations described above, values above/below predefined thresholds and/or having a standard deviation higher than a predefined threshold can be discarded.

Lastly, in step 532, the first initial position of the contact point C (cf. FIG. 14) of the marker is determined along axis v connecting the center of symmetry $C_m$ of the main body 12 of the marker, determined in block 528, and the lower vertex $V_{low}$, determined in block 530. In particular, the contact point C is set along such axis v at the known distance $h_1 + h_2$ (cf. FIGs. 14-15), in which the distance $h_1$ depends on the size of the main body 12 of the marker, and the distance $h_2$ is the distance between the contact surface 46 of the marker and the proximal face 15 of the main body 12.

Once the contact point C is found in the first calibrated image, the corresponding first 2D coordinates $(X_{i1}, Z_{i1})$ in the absolute calibrated reference system $(x, y, z)$ can be simply "read" in the calibrated image, since every position within the images captured from by the camera 112 and acquired by the processor 114 are mapped in the absolute calibrated reference system $(x, y, z)$ as a result of the calibration operation carried out in block 504 described above.

Although block 510 and related sub-blocks have been described with reference to a marker 40, the same analysis applies, *mutatis mutandis,* to markers 10 illustrated in FIG. 1, taking into account a different distance $h_2$ between the proximal face 15 of the main body 12 and the contact surface of the base.

Moreover, although the description above in connection with FIGs. 13-17 has been made with reference only to block 510 of determining the first initial positions $(X_{i1}, Z_{i1})$ of contact points of markers in the first calibrated image, the same operations and the similar geometric analysis apply, *mutatis mutandis,* to block 512 of determining the second initial positions $(Y_{i2}, Z_{i2})$ of contact points of markers in the second calibrated image.

In particular, the same description made above applies to block 512 by changing "first calibrated image" into "second calibrated image", "first initial position" into "second initial position", "first relative reference system" into " second relative reference system", first 2D coordinates $(X_{i1}, Z_{i1})$ into second 2D coordinates $(Y_{i1}, Z_{i1})$, and so forth.

In addition, it should be underlined that although blocks 502-522 of the method 500 are illustrated and described in a sequence, the order of some blocks could be modified without departing from the scope of the invention.

For example, the calibration operation of block 504 could also be carried out after the uncalibrated image and the first and second calibrated images have all been acquired (blocks 502, 506 and 508), the order of acquisition of the three images being irrelevant.

Alternatively or in addition, each of blocks 510 and 512 of determining the first and second initial positions of the contact point of the markers could be carried out after the respective acquisition block 506, 508 of the first and second image.

Moreover, blocks 516-522, related to the operations of curve fitting, parameters estimation, display of the reconstructed curve and data storage, could be carried out in a different reciprocal order or even simultaneously.

Moreover, although the system 100 has been described as comprising a single computational device 113 comprising a processor 114, such system may comprise a second remote processor, part of a second remote computational device, located elsewhere.

In particular, in such embodiment (not illustrated) the processor 114 may be programmed to execute blocks 502-508 of method 500 of the invention and to further send the first and second calibrated images to the remote processor (not illustrated), which may be programmed to acquire the first and second calibrated images from the processor 114 and to further execute blocks 510-522 of the method 500.

The foregoing detailed description has set forth various embodiments of the devices, system and methods via the use of block diagrams, schematics, and examples. With particular reference to the method according to the invention, insofar as such block diagrams, schematics, and examples contain one or more functions and/or operations, it will be understood by those skilled in the art that each function and/or operation within such block diagrams, flowcharts, or examples can be implemented, individually and/or collectively, by a wide range of hardware, software, firmware, or virtually any combination thereof.

However, those skilled in the art will recognize that the embodiments of the method disclosed herein, in whole or in part, can be implemented as one or more computer programs running on one or more processors similar to processor 114 described above (e.g., as one or more programs running on one or more computer systems), as one or more programs running on one or more controllers (e.g., microcontrollers), as one or more programs running on one or more processors (e.g., microprocessors), as firmware, or as virtually any combination thereof, and that designing the circuitry and/or writing the code for the software and/or firmware would be well within the skill of one of ordinary skill in the art in light of this disclosure.

Those of skill in the art will recognize that many of the steps and algorithms set out herein may employ additional acts, may omit some acts, and/or may execute acts in a different order than specified.

In addition, those skilled in the art will appreciate that the mechanisms taught herein are capable of being distributed as a program product in a variety of forms, and that an illustrative embodiment applies equally regardless of the particular type of signal bearing media used to actually carry out the distribution. Examples of signal bearing media include, but are not limited to, the following: recordable type media such as hard disk drives, CD ROMs, digital tape, and computer memory.

**Claims**

1. Method (500) for obtaining a spatial pattern of an anatomical structure of a subject (H), comprising the steps of:

   a) acquiring, from at least one digital image capturing device (112), at least one uncalibrated image of a calibration reference (CRef) applied on a surface (118) configured to receive the subject (H) in standing position thereon, said calibration reference (CRef) having at least one known dimension and defining at least one known direction;
   b) defining an absolute calibrated reference system of coordinates (x, y, z) based on the calibration reference (CRef) depicted in said at least one uncalibrated image; and
   c) acquiring, from said at least one digital image capturing device (112), at least a first and at least a second calibrated image of a plurality (110) of markers applied on a corresponding plurality of body landmarks (50) of the anatomical structure of the subject (H) at respective contact points (C) with the body landmarks (50), said plurality (110) of markers being arranged within said absolute calibrated reference system (x, y, z);
   wherein said at least one uncalibrated image acquired in step a), and said at least a first and said at least a second calibrated image acquired in step c), are captured by the at least one digital image capturing device (112) with the focal axis (F) thereof substantially parallel to said surface (118) configured to receive the subject (H);

wherein said at least a first calibrated image depicts a marker spatial arrangement in a first plane (x, z) and said at least a second calibrated image depicts the marker spatial arrangement in a second plane (y, z) different from the first plane (x, z), the first and second planes (x, z; y, z) being representative of a relative orientation of said plurality (110) of markers with respect to the at least one digital image capturing device (112);

wherein said markers (10, 40) of said plurality (110) of markers each comprise a respective three-dimensional main body (12) shaped as a polyhedron of N faces and having N-1 exposed faces (18) arranged in a known geometric relationship with the contact point (C) of the marker (10, 40) with the respective body landmark (50); and

wherein at least one of said N-1 exposed faces (18) of each marker (10, 40) is identifiable in said at least first and second calibrated images by means of an image recognition algorithm,

the method further comprising the step of:

d) determining for each marker (10, 40) of said plurality (110) of markers a position $(X_i, Y_i, Z_i)$ of the contact point (C) with the respective body landmark (50) of the subject (H) within said absolute calibrated reference system (x, y, z).

2. Method (500) according to any one of the preceding claims, wherein the first plane (x, z) and the second plane (y, z) are perpendicular to each other and preferably share a common vertical coordinate (z),

wherein more preferably said first plane (x, z) of the first calibrated image substantially coincides with a coronal plane of the subject (H) in standing position on the surface (118), and said second plane (y, z) of the second calibrated image substantially coincides with a sagittal plane of the subject (H) in standing position on the surface (118).

3. Method (500) according to any one of the preceding claims, wherein the main body (12) of each marker (10, 40) of said plurality (110) of markers is shaped as a regular polyhedron.

4. Method (500) according to any one of the preceding claims, wherein said step d) comprises:

d1) determining for each marker (10, 40) of the plurality (110) of markers a first initial position $(X_{i1}, Z_{i1})$ of the respective contact point (C) of the marker (10, 40) in the first calibrated image acquired in step c),

d2) determining for each marker (10, 40) of the plurality (110) of markers a second initial position $(Y_{i2}, Z_{i2})$ of the respective contact point (C) of the marker (10, 40) in the second calibrated image acquired in step c), and

d3) combining the first and second initial positions $(X_{i1}, Z_{i1}; Y_{i2}, Z_{i2})$, respectively determined in steps d1) and d2) from said first and second calibrated images, to obtain the position $(X_i, Y_i, Z_i)$ of each marker, preferably by averaging a common axial coordinate (Zi1, Zi2) between said first and said second initial positions (Xi1, Zi1; Yi1, Zi2) respectively determined from said at least a first and said at least a second calibrated images.

5. Method (500) according to claim 4, wherein said steps d1) and d2) each comprise, for each marker (10, 40) of the plurality (110) of markers depicted in said at least a first, respectively said at least a second, calibrated image:

d4) identifying one or more exposed faces (18) of the marker (10, 40) by recognizing a respective distinctive feature thereof;

d5) determining in said at least a first calibrated image, respectively, in said at least a second calibrated image, based on an orientation of the one or more exposed faces (18) of the marker (10, 40) recognized, a position of a center of symmetry $(C_m)$ of the main body (12) of the marker (10, 40);

d6) determining in said at least a first calibrated image, respectively, in said at least a second calibrated image, based on an orientation of the one or more exposed faces (18) of the marker (10, 40) recognized, the direction of an axis of symmetry (v) of the marker (10, 40) passing through a contact surface (16, 46) of the marker (10, 40) with the respective body landmark (50);

d7) determining in said at least a first calibrated image, respectively, in said at least a second calibrated image, the first initial position $(X_{i1}, Z_{i1})$, respectively, the second initial position $(Y_{i2}, Z_{i2})$, of the contact point (C) of the marker (10, 40) along said axis of symmetry (v) of the marker (10, 40), at a known distance from the center of symmetry $(C_m)$ of the main body (12) of the marker (10, 40),

wherein said step d) preferably comprises defining a relative reference system of coordinates $(w_1, w_2, w_3)$ for each distinctive feature recognized, said relative reference system of coordinates $(w_1, w_2, w_3)$ being centered at a center of symmetry of the distinctive feature recognized.

6. Method (500) according to any one of the preceding claims, further comprising the step of:

e) fitting the positions $(X_i, Y_i, Z_i)$ of the contact points (C) of markers (10, 40) of said plurality (110) of markers with a curve (RC), preferably by means of an interpolation operation selected from polynomial interpolation and spline interpolation, more preferably by means of cubic spline interpolation;

and/or the step of:

f) calculating, from the curve (RC) fitted in step e), parameters of interest related to the anatomical structure of the skeleton of the subject (H), and/or displaying said curve (RC), said parameters of interest calculated in step f) preferably being selected among distances, areas, angles, curvatures.

7. Method (500) according to any one of the preceding claims, wherein said surface (118) has a first reference plane ($t_1$) substantially coinciding, in use, with a coronal plane of the subject (H) standing thereon, and a second reference plane ($t_2$) substantially coinciding, in use, with a sagittal plane of the subject (H) standing thereon,

wherein said first plane (x, z) of the first calibrated image is preferably substantially parallel to or coincides with the first reference plane ($t_1$) of the surface (118), and said second plane (y, z) of the second calibrated image is preferably substantially parallel to or coincides with the second reference plane ($t_2$) of the surface (118), and wherein said at least one uncalibrated image acquired in step a), and said at least a first and said at least a second calibrated image acquired in step c), are captured by the at least one digital image capturing device (112) with a lens (127) thereof positioned at a same fixed distance (D) along the focal axis (F) from either the first reference plane ($t_1$) or the second reference plane ($t_2$) of said surface (118).

8. System (100) for obtaining a spatial pattern of an anatomical structure of a subject (H), said system comprising:

- a calibration reference (CRef) applied on a surface (118) configured to receive the subject (H), said calibration reference (CRef) having at least one known dimension and defining at least one known direction;
- a plurality of three-dimensional markers (110) configured to be applied on a corresponding plurality of body landmarks (50) of the anatomical structure of the subject (H) at respective contact points (C), each marker (10, 40) of said plurality of markers (110) comprising a respective three-dimensional main body (12), shaped as a polyhedron of N faces, and having N-1 exposed faces (18) arranged in a known geometric relationship with the contact point (C) of the marker (10, 40) with the respective body landmark (50), wherein at least one of said N-1 exposed faces (18) of each marker of said plurality of markers (110) is identifiable, by means of an image recognition algorithm, in an image of the marker (10, 40) captured by a digital image capturing device (112);

and

- at least one digital image capturing device (112) configured to:

(i) capture at least one uncalibrated image of said calibration reference (CRef); and
(ii) capture at least a first and at least a second calibrated image of said plurality (110) of markers applied on said corresponding plurality of body landmarks (50) and arranged within said absolute calibrated reference system (x, y, z) defined based on said at least one uncalibrated image of the calibration reference (CRef),

wherein said at least a first calibrated image depicts a marker spatial arrangement in a first plane (x, z) and said at least a second calibrated image depicts the marker spatial arrangement in a second plane (y, z) different from the first plane (x, z), the first and second planes (x, z; y, z) being representative of a relative orientation of said plurality (110) of markers with respect to the at least one digital image capturing device (112), wherein the system further comprises

- a processor (114) programmed to:

(i) acquire said at least one uncalibrated image of the calibration reference (CRef) from the at least one digital image capturing device (112);
(ii) define said absolute calibrated reference system of coordinates (x, y, z) based on said at least one uncalibrated image;
(iii) acquire said at least a first and at least a second calibrated images from the at least one digital image capturing device (112); and
(iv) determine for each marker of said plurality of markers (110) a position ($X_i$, $Y_i$, $Z_i$) of the contact point (C) with the respective body landmark (50) of the subject (H) within said absolute calibrated reference system (x, y, z),

wherein said at least one acquired uncalibrated image and said at least a first and said at least a second calibrated image are captured by the at least one digital image capturing device (112) with the focal axis (F) thereof substantially parallel to said surface (118) configured to receive the subject (H).

9. System (100) according to claim 8, further comprising:

- a first processor (114) programmed to:

(i) acquire said at least one uncalibrated image of the calibration reference (CRef) from the at least one digital image capturing device (112);
(ii) define said absolute calibrated reference system of coordinates (x, y, z) based on said at least one uncalibrated image;
(iii) acquire said at least a first and said at least a second calibrated images from the at least one digital image capturing device (112); and
(iii_bis) transmit said at least a first and at least a second calibrated images of said plurality (110) of markers to a second processor;

and
- the second processor (114) programmed to:

(iii_ter) acquire said at least a first and said at least a second calibrated images of said plurality (110) of markers from said first processor; and
(iv) determine for each marker of said plurality of markers the position $(X_i, Y_i, Z_i)$ of the contact point (C) with the respective body landmark (50) of the subject (H) within said absolute calibrated reference system (x, y, z), said second processor optionally being remote with respect to said first processor.

10. System (100) according to any one of claims 8 or 9, wherein the main body (12) of each marker (10, 40) of said plurality (110) of markers is shaped as a regular polyhedron.

11. System (100) according to any one of claims 8 to 10, further comprising:

- a support (116) having said calibration reference (CRef) positioned on an upper surface (118) thereof, said support (116) being configured to receive the subject (H) in standing position,
wherein preferably said support (116) is rotatably mounted onto a stationary stand configured to be laid on the ground, said support (116) being preferably rotatable between fixed angular positions angularly spaced of 90°, wherein even more preferably said support (116) comprises a guide (122, 222) configured to set up an angle ($\alpha$) between feet of the subject (H) positioned thereon.

12. System (100) according to any one of claims 8-11, wherein said surface (118) has a first reference plane ($t_1$) substantially coinciding, in use, with a coronal plane of the subject (H) standing thereon, and a second reference plane ($t_2$) substantially coinciding, in use, with a sagittal plane of the subject (H) standing thereon.

13. System (100) according to claim 12, wherein said first and second reference planes ($t_1$, $t_2$) of the surface (118) are median planes of the support (116).

14. System according to any one of claims 8 to 13, wherein each marker (10, 40) is configured to obtain a spatial pattern of an anatomical structure of a subject (H), said marker being configured to be applied on a body landmark (50) of the anatomical structure of the subject (H) at a respective contact point (C) and comprising a three-dimensional main body (12) shaped as a polyhedron of N faces, having N-1 exposed faces (18) arranged in a known geometric relationship with the contact point (C) of the marker (10, 40) with the respective body landmark (50);
wherein at least one of said N-1 exposed faces (18) of the marker (10, 40) is identifiable, by means of an image recognition algorithm, in an image of the marker (10, 40) captured by a digital image capturing device.

15. System (10, 40) according to claim 14, wherein the main body (12) has a shape selected from an octahedron, a dodecahedron, icosahedron, preferably being a dodecahedron.

**Patentansprüche**

1. Verfahren (500) zur Gewinnung eines räumlichen Musters einer anatomischen Struktur eines Subjekts (H), das die folgenden Schritte umfasst:

a) Erfassen mindestens eines unkalibrierten Bildes einer Kalibrierungsreferenz (CRef), die auf einer Oberfläche (118) angebracht ist, die so konfiguriert ist, dass sie das Subjekt (H) in stehender Position aufnimmt, von mindestens einer digitalen Bilderfassungsvorrichtung (112), wobei die Kalibrierungsreferenz (CRef) mindestens eine bekannte Abmessung aufweist und mindestens eine bekannte Richtung definiert;

b) Definieren eines absoluten kalibrierten Bezugssystems von Koordinaten (x, y, z) auf der Grundlage der Kalibrierungsreferenz (CRef), die in dem mindestens einen unkalibrierten Bild dargestellt ist; und

c) Erfassen mindestens eines ersten und mindestens eines zweiten kalibrierten Bildes einer Vielzahl (110) von Markierungen, die an einer entsprechenden Vielzahl von Körpermerkmalen (50) der anatomischen Struktur des Subjekts (H) an jeweiligen Kontaktpunkten (C) mit den Körpermerkmalen (50) angebracht sind, von der mindestens einen digitalen Bilderfassungsvorrichtung (112), wobei die Vielzahl (110) von Markierungen innerhalb des absoluten kalibrierten Referenzsystems (x, y, z) angeordnet ist;

wobei das mindestens eine unkalibrierte Bild, das in Schritt a) erfasst wurde, und das mindestens erste und das mindestens zweite kalibrierte Bild, die in Schritt c) erfasst wurden, von der mindestens einen digitalen Bilderfassungsvorrichtung (112) erfasst werden, deren Brennachse (F) im Wesentlichen parallel zu der Oberfläche (118) verläuft, die zur Aufnahme des Objekts (H) konfiguriert ist;

wobei das mindestens erste kalibrierte Bild eine räumliche Anordnung von Markierungen in einer ersten Ebene (x, z) darstellt und das mindestens zweite kalibrierte Bild die räumliche Anordnung von Markierungen in einer zweiten Ebene (y, z) darstellt, die sich von der ersten Ebene (x, z) unterscheidet, wobei die erste und die zweite Ebene (x, z; y, z) für eine relative Ausrichtung der Vielzahl (110) von Markierungen in Bezug auf die mindestens eine digitale Bilderfassungsvorrichtung (112) repräsentativ sind;

wobei die Markierungen (10, 40) der Vielzahl (110) von Markierungen jeweils einen jeweiligen dreidimensionalen Hauptkörper (12) umfassen, der als Polyeder mit N Flächen geformt ist und N-1 freiliegende Flächen (18) aufweist, die in einer bekannten geometrischen Beziehung zu dem Kontaktpunkt (C) der Markierung (10, 40) mit der jeweiligen Körperlandmarke (50) angeordnet sind; und

wobei mindestens eine der N-1 belichteten Flächen (18) jeder Markierung (10, 40) in den mindestens ersten und zweiten kalibrierten Bildern mit Hilfe eines Bilderkennungsalgorithmus identifizierbar ist,

wobei das Verfahren ferner den folgenden Schritt umfasst:

d) Bestimmen einer Position $(X_i, Y_i, Z_i)$ des Kontaktpunktes (C) mit dem jeweiligen Körpermerkmal (50) des Subjekts (H) innerhalb des absoluten kalibrierten Bezugssystems (x, y, z) für jeden Marker (10, 40) der Vielzahl (110) von Markern.

2. Verfahren (500) nach einem der vorhergehenden Ansprüche, wobei die erste Ebene (x, z) und die zweite Ebene (y, z) senkrecht zueinander stehen und vorzugsweise eine gemeinsame vertikale Koordinate (z) haben,

wobei vorzugsweise die erste Ebene (x, z) des ersten kalibrierten Bildes im Wesentlichen mit einer koronalen Ebene des Subjekts (H) in stehender Position auf der Oberfläche (118) übereinstimmt, und die zweite Ebene (y, z) des zweiten kalibrierten Bildes im Wesentlichen mit einer sagittalen Ebene des Subjekts (H) in stehender Position auf der Oberfläche (118) übereinstimmt.

3. Verfahren (500) nach einem der vorhergehenden Ansprüche, wobei der Hauptkörper (12) jedes Markers (10, 40) der Vielzahl (110) von Markern als ein regelmäßiges Polyeder geformt ist.

4. Verfahren (500) nach einem der vorhergehenden Ansprüche, wobei der Schritt d) umfasst:

d1) Bestimmen einer ersten Anfangsposition $(X_{i1}, Z_{i1})$ des jeweiligen Kontaktpunktes (C) der Markierung (10, 40) in dem in Schritt c) erfassten ersten kalibrierten Bild für jede Markierung (10, 40) der Vielzahl (110) von Markierungen,

d2) Bestimmen einer zweiten Anfangsposition $(Y_{i2}, Z_{i2})$ des jeweiligen Kontaktpunkts (C) der Markierung (10, 40) in dem zweiten kalibrierten Bild, das in Schritt c) erfasst wurde, für jede Markierung (10, 40) der Vielzahl (110) von Markierungen, und

d3) Kombinieren der ersten und zweiten Anfangspositionen $(X_{i1}, Z_{i1}; Y_{i2}, Z_{i2})$, die jeweils in den Schritten d1) und d2) aus den ersten und zweiten kalibrierten Bildern bestimmt wurden, um die Position $(X_i, Y_i, Z_i)$ jeder Markierung zu erhalten, vorzugsweise durch Mittelung einer gemeinsamen axialen Koordinate $(Z_{i1}, Z_{i2})$ zwischen der ersten und der zweiten Anfangsposition $(X_{i1}, Z_{i1}; Y_{i1}, Z_{i2})$, die jeweils aus dem mindestens einen ersten und dem mindestens einen zweiten kalibrierten Bild bestimmt werden.

5. Verfahren (500) nach Anspruch 4, wobei die Schritte d1) und d2) jeweils für jeden Marker (10, 40) der Vielzahl (110) von Markern, die in dem mindestens ersten bzw. dem mindestens zweiten kalibrierten Bild dargestellt sind, umfassen:

d4) Identifizierung einer oder mehrerer freiliegender Flächen (18) des Markers (10, 40) durch Erkennung eines entsprechenden Unterscheidungsmerkmals;

d5) Bestimmen einer Position eines Symmetriezentrums ($C_m$) des Hauptkörpers (12) des Markers (10, 40) in dem mindestens ersten kalibrierten Bild bzw. in dem mindestens zweiten kalibrierten Bild auf der Grundlage einer Ausrichtung der einen oder mehreren erkannten exponierten Flächen (18) des Markers (10, 40);

d6) Bestimmen der Richtung einer Symmetrieachse (v) des Markers (10, 40), die durch eine Kontaktfläche (16, 46) des Markers (10, 40) mit dem jeweiligen Körpermerkmal (50) verläuft, in dem mindestens ersten kalibrierten Bild bzw. in dem mindestens zweiten kalibrierten Bild auf der Grundlage einer Ausrichtung der einen oder mehreren erkannten exponierten Flächen (18) des Markers (10, 40);

d7) Bestimmen der ersten Anfangsposition ($X_{i1}$, $Z_{i1}$) bzw. der zweiten Anfangsposition ($Y_{i2}$) des Kontaktpunkts (C) des Markers (10, 40) entlang der Symmetrieachse (v) des Markers (10, 40) in dem mindestens ersten kalibrierten Bild bzw. in dem mindestens zweiten kalibrierten Bild, $Z_{i2}$) des Kontaktpunktes (C) der Markierung (10, 40) entlang der Symmetrieachse (v) der Markierung (10, 40) in einem bekannten Abstand vom Symmetriezentrum ($C_m$) des Hauptkörpers (12) der Markierung (10, 40),

wobei der Schritt d) vorzugsweise das Definieren eines relativen Bezugssystems von Koordinaten ($w_1$, $w_2$, $w_3$) für jedes erkannte Unterscheidungsmerkmal umfaßt, wobei das relative Bezugssystem von Koordinaten ($w_1$, $w_2$, $w_3$) auf ein Symmetriezentrum des erkannten Unterscheidungsmerkmals zentriert ist.

6. Verfahren nach einem der vorhergehenden Ansprüche, das ferner die folgenden Schritte umfasst:

e) Anpassen der Positionen ($X_i$, $Y_i$, $Z_i$) der Kontaktpunkte (C) von Markierungen (10, 40) der Vielzahl (110) von Markierungen an eine Kurve (RC), vorzugsweise mittels einer Interpolationsoperation, die aus der Polynominterpolation und der Spline-Interpolation ausgewählt wird, besonders bevorzugt mittels der kubischen Spline-Interpolation;
und/oder den Schritt von:

f) Berechnen von interessierenden Parametern, die mit der anatomischen Struktur des Skeletts des Probanden (H) zusammenhängen, aus der in Schritt e) angepassten Kurve (RC) und/oder Anzeigen der Kurve (RC), wobei die in Schritt f) berechneten interessierenden Parameter vorzugsweise unter Abständen, Flächen, Winkeln, Krümmungen ausgewählt werden.

7. Verfahren (500) nach einem der vorhergehenden Ansprüche, wobei die Oberfläche (118) eine erste Bezugsebene ($t_1$) aufweist, die im Gebrauch im Wesentlichen mit einer Koronalebene des darauf stehenden Subjekts (H) zusammenfällt, und eine zweite Bezugsebene ($t_2$), die im Gebrauch im Wesentlichen mit einer Sagittalebene des darauf stehenden Subjekts (H) zusammenfällt,

wobei die erste Ebene (x, z) des ersten kalibrierten Bildes vorzugsweise im wesentlichen parallel zu der ersten Referenzebene ($t_1$) der Oberfläche (118) ist oder mit dieser zusammenfällt, und die zweite Ebene (y, z) des zweiten kalibrierten Bildes vorzugsweise im wesentlichen parallel zu der zweiten Referenzebene ($t_2$) der Oberfläche (118) ist oder mit dieser zusammenfällt, und

wobei das mindestens eine unkalibrierte Bild, das in Schritt a) erfasst wurde, und das mindestens eine erste und das mindestens eine zweite kalibrierte Bild, die in Schritt c) erfasst wurden, von der mindestens einen digitalen Bilderfassungsvorrichtung (112) erfasst werden, deren Objektiv (127) in demselben festen Abstand (D) entlang der Brennachse (F) entweder von der ersten Referenzebene ($t_1$) oder der zweiten Referenzebene ($t_2$) der Oberfläche (118) positioniert ist.

8. System (100) zum Erhalten eines räumlichen Musters einer anatomischen Struktur eines Subjekts (H), wobei das System umfasst:

- eine Kalibrierungsreferenz (CRef), die auf einer Oberfläche (118) angebracht ist, die so konfiguriert ist, dass sie das Objekt (H) aufnimmt, wobei die Kalibrierungsreferenz (CRef) mindestens eine bekannte Abmessung aufweist und mindestens eine bekannte Richtung definiert;

- eine Vielzahl von dreidimensionalen Markierungen (110), die so konfiguriert sind, dass sie an einer entsprechenden Vielzahl von Körpermerkmalen (50) der anatomischen Struktur des Subjekts (H) an jeweiligen Kontaktpunkten (C) angebracht werden können, wobei jede Markierung (10, 40) der Vielzahl von Markierungen (110) einen jeweiligen dreidimensionalen Hauptkörper (12) umfasst, der als ein Polyeder mit N Flächen geformt ist und N-1 freiliegende Flächen (18) aufweist, die in einer bekannten geometrischen Beziehung zu dem Kontaktpunkt (C) der Markierung (10, 40) mit dem jeweiligen Körpermerkmal (50) angeordnet sind,
wobei mindestens eine der N-1 belichteten Flächen (18) jeder Markierung der Vielzahl von Markierungen (110) mittels eines Bilderkennungsalgorithmus in einem Bild der Markierung (10, 40) identifizierbar ist, das von einer digitalen Bilderfassungsvorrichtung (112) erfasst wurde;
und
- mindestens eine digitale Bilderfassungsvorrichtung (112), die dafür konfiguriert ist:

(i) Erfassung mindestens eines unkalibrierten Bildes der Kalibrierungsreferenz (CRef); und
(ii) Erfassen mindestens eines ersten und mindestens eines zweiten kalibrierten Bildes der Vielzahl (110) von Markierungen, die auf der entsprechenden Vielzahl von Körpermerkmalen (50) angebracht und innerhalb des absoluten kalibrierten Referenzsystems (x, y, z) angeordnet sind, das auf der Grundlage des mindestens einen unkalibrierten Bildes der Kalibrierungsreferenz (CRef) definiert ist,

wobei das mindestens erste kalibrierte Bild eine räumliche Anordnung von Markern in einer ersten Ebene (x, z) darstellt und das mindestens zweite kalibrierte Bild die räumliche Anordnung von Markern in einer zweiten Ebene (y, z) darstellt, die sich von der ersten Ebene (x, z) unterscheidet, wobei die erste und die zweite Ebene (x, z; y, z) für eine relative Ausrichtung der Vielzahl (110) von Markern in Bezug auf die mindestens eine digitale Bilderfassungsvorrichtung (112) repräsentativ sind, wobei das System ferner umfasst
- einen Prozessor (114), der so programmiert ist, dass er:

(i) das mindestens eine unkalibrierte Bild der Kalibrierungsreferenz (CRef) von der mindestens einen digitalen Bilderfassungsvorrichtung (112) zu erfassen;
(ii) das absolute kalibrierte Koordinatensystem (x, y, z) auf der Grundlage des mindestens einen unkalibrierten Bildes zu definieren;
(iii) Erfassen mindestens eines ersten und mindestens eines zweiten kalibrierten Bildes von der mindestens einen digitalen Bilderfassungsvorrichtung (112); und
(iv) Bestimmen einer Position ($X_i$, $Y_i$, $Z_i$) des Kontaktpunktes (C) mit dem jeweiligen Körpermerkmal (50) des Subjekts (H) innerhalb des absoluten kalibrierten Bezugssystems (x, y, z) für jeden Marker der Vielzahl von Markern (110),

wobei das mindestens eine erfasste unkalibrierte Bild und das mindestens eine erste und das mindestens eine zweite kalibrierte Bild von der mindestens einen digitalen Bilderfassungsvorrichtung (112) erfasst werden, deren Brennachse (F) im Wesentlichen parallel zu der Oberfläche (118) verläuft, die zur Aufnahme des Objekts (H) konfiguriert ist.

9. System (100) nach Anspruch 8, ferner umfassend:

- einen ersten Prozessor (114), der so programmiert ist, dass er:

(i) das mindestens eine unkalibrierte Bild der Kalibrierungsreferenz (CRef) von der mindestens einen digitalen Bilderfassungsvorrichtung (112) zu erfassen;
(ii) das absolute kalibrierte Koordinatensystem (x, y, z) auf der Grundlage des mindestens einen unkalibrierten Bildes zu definieren;
(iii) Erfassen des mindestens einen ersten und des mindestens einen zweiten kalibrierten Bildes von der mindestens einen digitalen Bilderfassungsvorrichtung (112); und
(iii_bis) Übertragen der mindestens ersten und mindestens zweiten kalibrierten Bilder der Vielzahl (110) von Markierungen an einen zweiten Prozessor;

und
- der zweite Prozessor (114) ist programmiert, um:

(iii_ter) Erfassen des mindestens ersten und des mindestens zweiten kalibrierten Bildes der Vielzahl (110) von Markern von dem ersten Prozessor; und
(iv) Bestimmen der Position ($X_i$, $Y_i$, $Z_i$) des Kontaktpunktes (C) mit dem jeweiligen Körpermerkmal (50) des Subjekts (H) innerhalb des absoluten kalibrierten Bezugssystems (x, y, z) für jeden Marker der Vielzahl von Markern,
wobei der zweite Prozessor optional von dem ersten Prozessor entfernt sein kann.

10. System (100) nach einem der Ansprüche 8 oder 9, wobei der Hauptkörper (12) jedes Markers (10, 40) der Vielzahl (110) von Markern die Form eines regelmäßigen Polyeders hat.

11. Überwachungssystem nach einem der Ansprüche 8 bis 10, ferner Folgendes umfassend:

- einen Träger (116), auf dessen Oberseite (118) die Kalibrierungsreferenz (CRef) positioniert ist, wobei der Träger (116) so konfiguriert ist, dass er das Subjekt (H) in stehender Position aufnimmt, wobei vorzugsweise der Träger (116) drehbar auf einem stationären Ständer montiert ist, der so konfiguriert ist, dass er auf den Boden gelegt werden kann, wobei der Träger (116) vorzugsweise zwischen festen Winkelpositionen drehbar ist, die in einem Winkelabstand von 90° angeordnet sind, wobei noch mehr bevorzugt der Träger (116) eine Führung (122, 222) umfasst, die so konfiguriert ist, dass sie einen Winkel (a) zwischen den Füßen des darauf positionierten Subjekts (H) einstellt.

12. System (100) nach einem der Ansprüche 8-11, wobei die Oberfläche (118) eine erste Bezugsebene ($t_1$) aufweist, die im Gebrauch im Wesentlichen mit einer Koronalebene des darauf stehenden Subjekts (H) übereinstimmt, und eine zweite Bezugsebene ($t_2$), die im Gebrauch im Wesentlichen mit einer Sagittalebene des darauf stehenden Subjekts (H) übereinstimmt.

13. System (100) nach Anspruch 12, wobei die erste und zweite Bezugsebene ($t_1$, $t_2$) der Oberfläche (118) Mittelebenen des Trägers (116) sind.

14. System nach einem der Ansprüche 8 bis 13, wobei jeder Marker (10, 40) so konfiguriert ist, dass er ein räumliches Muster einer anatomischen Struktur eines Subjekts (H) erhält, wobei der Marker so konfiguriert ist, dass er auf einem Körpermerkmal (50) der anatomischen Struktur des Subjekts (H) an einem jeweiligen Kontaktpunkt (C) angebracht wird, und einen dreidimensionalen Hauptkörper (12) umfasst, der als Polyeder mit N Flächen geformt ist, mit N-1 freiliegenden Flächen (18), die in einer bekannten geometrischen Beziehung zu dem Kontaktpunkt (C) des Markers (10, 40) mit dem jeweiligen Körpermerkmal (50) angeordnet sind; wobei mindestens eine der N-1 belichteten Flächen (18) der Markierung (10, 40) mittels eines Bilderkennungsalgorithmus in einem Bild der Markierung (10, 40) identifizierbar ist, das von einer digitalen Bilderfassungsvorrichtung aufgenommen wurde.

15. System (10, 40) nach Anspruch 14, wobei der Hauptkörper (12) eine Form hat, die aus einem Oktaeder, einem Dodekaeder und einem Ikosaeder ausgewählt ist, wobei er vorzugsweise ein Dodekaeder ist.

**Revendications**

1. Méthode (500) pour obtenir un schéma spatial d'une structure anatomique d'un sujet (H), comprenant les étapes suivantes :

a) acquisition, à partir d'au moins un dispositif de capture d'images numériques (112), d'au moins une image non calibrée d'une référence de calibrage (CRef) appliquée sur une surface (118) configurée pour recevoir le sujet (H) en position debout sur celle-ci, ladite référence de calibrage (CRef) ayant au moins une dimension connue et définissant au moins une direction connue ;

b) définir un système de référence calibré absolu de coordonnées (x, y, z) basé sur la référence de calibrage (CRef) représentée dans ladite au moins une image non calibrée ; et

c) acquérir, à partir dudit au moins un dispositif de capture d'images numériques (112), au moins une première et au moins une deuxième image calibrée d'une pluralité (110) de marqueurs appliqués sur une pluralité correspondante de repères corporels (50) de la structure anatomique du sujet (H) à des points de contact respectifs (C) avec les repères corporels (50), ladite pluralité (110) de marqueurs étant disposée dans ledit système de référence calibré absolu (x, y, z) ;

dans laquelle ladite au moins une image non calibrée acquise à l'étape a), et ladite au moins une première et ladite au moins une seconde image calibrée acquises à l'étape c), sont capturées par l'au moins un dispositif de capture d'images numériques (112) dont l'axe focal (F) est sensiblement parallèle à ladite surface (118) configurée pour recevoir le sujet (H) ;

dans laquelle au moins une première image calibrée représente une disposition spatiale du marqueur dans un premier plan (x, z) et au moins une deuxième image calibrée représente la disposition spatiale du marqueur dans un deuxième plan (y, z) différent du premier plan (x, z), le premier et le deuxième plan (x, z ; y, z) étant représentatifs d'une orientation relative de ladite pluralité (110) de marqueurs par rapport à l'au moins un dispositif de capture d'images numériques (112) ;

dans lequel lesdits marqueurs (10, 40) de ladite pluralité (110) de marqueurs comprennent chacun un corps principal tridimensionnel respectif (12) ayant la forme d'un polyèdre à N faces et comportant N-1 faces exposées (18) disposées dans une relation géométrique connue avec le point de contact (C) du marqueur (10, 40) avec le repère corporel respectif (50) ; et

dans lequel au moins une des N-1 faces exposées (18) de chaque marqueur (10, 40) est identifiable dans les première et deuxième images calibrées au moyen d'un algorithme de reconnaissance d'images,

la méthode comprend en outre l'étape suivante :

d) déterminer pour chaque marqueur (10, 40) de ladite pluralité (110) de marqueurs une position $(X_i, Y_i, Z_i)$ du point de contact (C) avec le repère corporel respectif (50) du sujet (H) dans ledit système de référence calibré absolu (x, y, z).

2. Méthode (500) selon l'une quelconque des revendications précédentes, dans laquelle le premier plan (x, z) et le second plan (y, z) sont perpendiculaires l'un à l'autre et partagent de préférence une coordonnée verticale commune (z),

de préférence, le premier plan (x, z) de la première image calibrée coïncide sensiblement avec un plan coronal du sujet (H) en position debout sur la surface (118), et le second plan (y, z) de la seconde image calibrée coïncide sensiblement avec un plan sagittal du sujet (H) en position debout sur la surface (118).

3. Procédé (500) selon l'une quelconque des revendications précédentes, dans lequel le corps principal (12) de chaque marqueur (10, 40) de ladite pluralité (110) de marqueurs a la forme d'un polyèdre régulier.

4. Procédé (500) selon l'une quelconque des revendications précédentes, dans lequel ladite étape d) comprend :

d1) déterminer pour chaque marqueur (10, 40) de la pluralité (110) de marqueurs une première position initiale $(X_{i1}, Z_{i1})$ du point de contact respectif (C) du marqueur (10, 40) dans la première image calibrée acquise à l'étape c),

d2) déterminer pour chaque marqueur (10, 40) de la pluralité (110) de marqueurs une deuxième position initiale $(Y_{i2}, Z_{i2})$ du point de contact respectif (C) du marqueur (10, 40) dans la deuxième image calibrée acquise à l'étape c), et

d3) combiner les première et deuxième positions initiales $(X_{i1}, Z_{i1}; Y_{i2}, Z_{i2})$, respectivement déterminées aux étapes d1) et d2) à partir desdites première et deuxième images calibrées, pour obtenir la position $(X_i, Y_i, Z_i)$ de chaque marqueur, de préférence en calculant la moyenne d'une coordonnée axiale commune $(Z_{i1}, Z_{i2})$ entre lesdites première et deuxième positions initiales $(X_{i1}, Z_{i1}; Y_{i1}, Z_{i2})$ respectivement déterminées à partir desdites au moins une première et au moins une deuxième images calibrées.

5. Procédé (500) selon la revendication 4, dans lequel lesdites étapes d1) et d2) comprennent chacune, pour chaque marqueur (10, 40) de la pluralité (110) de marqueurs représentés dans ladite au moins une première, respectivement ladite au moins une seconde, image calibrée :

d4) identifier une ou plusieurs faces exposées (18) du marqueur (10, 40) en reconnaissant leurs caractéristiques distinctives respectives ;

d5) déterminer dans ladite au moins une première image calibrée, respectivement, dans ladite au moins une deuxième image calibrée, sur la base d'une orientation des une ou plusieurs faces exposées (18) du marqueur (10, 40) reconnu, une position d'un centre de symétrie $(C_m)$ du corps principal (12) du marqueur (10, 40) ;

d6) déterminer dans ladite au moins une première image calibrée, respectivement dans ladite au moins une deuxième image calibrée, sur la base d'une orientation des une ou plusieurs faces exposées (18) du marqueur (10, 40) reconnu, la direction d'un axe de symétrie (v) du marqueur (10, 40) passant par une surface de contact (16, 46) du marqueur (10, 40) avec le point de repère corporel respectif (50) ;

d7) déterminer dans ladite au moins une première image calibrée, respectivement, dans ladite au moins une deuxième image calibrée, la première position initiale $(X_{i1}, Z_{i1})$, respectivement, la deuxième position initiale $(Y_{i2}, Z_{i2})$, du point de contact (C) du marqueur (10, 40) le long dudit axe de symétrie (v) du marqueur (10, 40), à une distance connue du centre de symétrie $(C_m)$ du corps principal (12) du marqueur (10, 40),

dans laquelle ladite étape d) consiste de préférence à définir un système de référence relatif de coordonnées $(w_1, w_2, w_3)$ pour chaque élément distinctif reconnu, ledit système de référence relatif de coordonnées $(w_1, w_2, w_3)$ étant centré sur un centre de symétrie de l'élément distinctif reconnu.

6. Procédé selon l'une quelconque des revendications précédentes, comprenant en outre les étapes suivantes :

e) adapter les positions $(X_i, Y_i, Z_i)$ des points de contact (C) des marqueurs (10, 40) de ladite pluralité (110) de marqueurs à une courbe (RC), de préférence au moyen d'une opération d'interpolation choisie parmi l'interpolation polynomiale et l'interpolation spline, plus préférablement au moyen d'une interpolation spline cubique ; et/ou l'étape suivante :

f) calculer, à partir de la courbe (RC) ajustée à l'étape e), des paramètres d'intérêt liés à la structure anatomique du squelette du sujet (H), et/ou afficher ladite courbe (RC), lesdits paramètres d'intérêt calculés à l'étape f) étant de préférence choisis parmi les distances, les surfaces, les angles, les courbures.

7. Procédé (500) selon l'une quelconque des revendications précédentes, dans lequel ladite surface (118) présente un premier plan de référence $(t_1)$ coïncidant sensiblement, en cours d'utilisation, avec un plan coronal du sujet (H) qui s'y tient debout, et un second plan de référence $(t_2)$ coïncidant sensiblement, en cours d'utilisation, avec un plan sagittal du sujet (H) qui s'y tient debout,

dans lequel ledit premier plan (x, z) de la première image calibrée est de préférence sensiblement parallèle au premier plan de référence $(t_1)$ de la surface (118) ou coïncide avec celui-ci, et ledit second plan (y, z) de la seconde image calibrée est de préférence sensiblement parallèle au second plan de référence $(t_2)$ de la surface (118) ou coïncide avec celui-ci, et

dans laquelle ladite au moins une image non calibrée acquise à l'étape a), et lesdites au moins une première et au moins une deuxième images calibrées acquises à l'étape c), sont capturées par l'au moins un dispositif de capture d'images numériques (112) avec un objectif (127) de celui-ci positionné à une même distance fixe (D) le long de l'axe focal (F) soit du premier plan de référence $(t_1)$ soit du deuxième plan de référence $(t_2)$ de ladite surface (118).

8. Système (100) pour obtenir un schéma spatial d'une structure anatomique d'un sujet (H), ledit système comprenant :

- une référence d'étalonnage (CRef) appliquée sur une surface (118) configurée pour recevoir le sujet (H), ladite référence d'étalonnage (CRef) ayant au moins une dimension connue et définissant au moins une direction connue ;
- une pluralité de marqueurs tridimensionnels (110) configurés pour être appliqués sur une pluralité correspondante de repères corporels (50) de la structure anatomique du sujet (H) à des points de contact respectifs (C), chaque marqueur (10, 40) de ladite pluralité de marqueurs (110) comprenant un corps principal tridimensionnel respectif (12), en forme de polyèdre à N faces, et ayant N-1 faces exposées (18) disposées dans une relation géométrique connue avec le point de contact (C) du marqueur (10, 40) avec le repère corporel respectif (50), dans lequel au moins une de ces N-1 faces exposées (18) de chaque marqueur de ladite pluralité de marqueurs (110) est identifiable, au moyen d'un algorithme de reconnaissance d'images, dans une image du marqueur (10, 40) capturée par un dispositif de capture d'images numériques (112) ; et
- au moins un dispositif de capture d'images numériques (112) configuré pour :

(i) capturer au moins une image non calibrée de ladite référence d'étalonnage (CRef) ; et
(ii) capturer au moins une première et au moins une deuxième image calibrée de ladite pluralité (110) de marqueurs appliqués sur ladite pluralité correspondante de repères corporels (50) et disposés dans ledit système de référence calibré absolu (x, y, z) défini sur la base de ladite au moins une image non calibrée de la référence d'étalonnage (CRef),

dans laquelle au moins une première image calibrée représente une disposition spatiale du marqueur dans un premier plan (x, z) et au moins une deuxième image calibrée représente la disposition spatiale du marqueur dans un deuxième plan (y, z) différent du premier plan (x, z), le premier et le deuxième plan (x, z ; y, z) étant représentatifs d'une orientation relative de ladite pluralité (110) de marqueurs par rapport à l'au moins un dispositif de capture d'images numériques (112), dans laquelle le système comprend en outre
- un processeur (114) programmé pour :

(i) acquérir ladite au moins une image non calibrée de la référence de calibrage (CRef) à partir d'au moins un dispositif de capture d'images numériques (112) ;
(ii) définir ledit système de référence calibré absolu de coordonnées (x, y, z) sur la base d'au moins une image non calibrée ;
(iii) acquérir lesdites au moins une première et au moins une deuxième images calibrées à partir d'au moins un dispositif de capture d'images numériques (112) ; et

(iv) déterminer pour chaque marqueur de ladite pluralité de marqueurs (110) une position $(X_i, Y_i, Z_i)$ du point de contact (C) avec le repère corporel respectif (50) du sujet (H) dans ledit système de référence calibré absolu (x, y, z),

dans laquelle ladite au moins une image non calibrée acquise et lesdites au moins une première et une seconde images calibrées sont capturées par l'au moins un dispositif de capture d'images numériques (112) dont l'axe focal (F) est sensiblement parallèle à ladite surface (118) configurée pour recevoir le sujet (H).

9. Système (100) selon la revendication 8, comprenant en outre :

- un premier processeur (114) programmé pour :

(i) acquérir ladite au moins une image non calibrée de la référence de calibrage (CRef) à partir d'au moins un dispositif de capture d'images numériques (112) ;
(ii) définir ledit système de référence calibré absolu de coordonnées (x, y, z) sur la base d'au moins une image non calibrée ;
(iii) acquérir lesdites au moins une première et au moins une deuxième images calibrées à partir d'au moins un dispositif de capture d'images numériques (112) ; et
(iii_bis) transmettre lesdites au moins une première et au moins une seconde images calibrées de ladite pluralité (110) de marqueurs à un second processeur ;

et
- le second processeur (114) programmé pour :

(iii_ter) acquérir lesdites au moins une première et ladite au moins une deuxième images calibrées de ladite pluralité (110) de marqueurs à partir dudit premier processeur ; et
(iv) déterminer pour chaque marqueur de ladite pluralité de marqueurs la position $(X_i, Y_i, Z_i)$ du point de contact (C) avec le repère corporel respectif (50) du sujet (H) dans ledit système de référence calibré absolu (x, y, z),
Ce deuxième processeur peut éventuellement être distant par rapport au premier processeur.

10. Système (100) selon l'une quelconque des revendications 8 ou 9, dans lequel le corps principal (12) de chaque marqueur (10, 40) de ladite pluralité (110) de marqueurs a la forme d'un polyèdre régulier.

11. Système de surveillance selon l'une quelconque des revendications 8 à 10, comprenant en outre :

- un support (116) ayant ladite référence d'étalonnage (CRef) positionnée sur une surface supérieure (118) de celui-ci, ledit support (116) étant configuré pour recevoir le sujet (H) en position debout,
dans lequel, de préférence, ledit support (116) est monté rotatif sur un support fixe configuré pour être posé sur le sol, ledit support (116) étant de préférence rotatif entre des positions angulaires fixes espacées de 90°,
de préférence, ledit support (116) comprend un guide (122, 222) configuré pour établir un angle (a) entre les pieds du sujet (H) positionné sur celui-ci.

12. Système (100) selon l'une quelconque des revendications 8 à 11, dans lequel ladite surface (118) présente un premier plan de référence $(t_1)$ coïncidant sensiblement, en cours d'utilisation, avec un plan coronal du sujet (H) se tenant sur cette surface, et un second plan de référence $(t_2)$ coïncidant sensiblement, en cours d'utilisation, avec un plan sagittal du sujet (H) se tenant sur cette surface.

13. Système (100) selon la revendication 12, dans lequel lesdits premier et deuxième plans de référence $(t_1, t_2)$ de la surface (118) sont des plans médians du support (116).

14. Système selon l'une quelconque des revendications 8 à 13, dans lequel chaque marqueur (10, 40) est configuré pour obtenir un motif spatial d'une structure anatomique d'un sujet (H), ledit marqueur étant configuré pour être appliqué sur un repère corporel (50) de la structure anatomique du sujet (H) à un point de contact respectif (C) et comprenant un corps principal tridimensionnel (12) ayant la forme d'un polyèdre à N faces, ayant N-1 faces exposées (18) disposées dans une relation géométrique connue avec le point de contact (C) du marqueur (10, 40) avec le repère corporel respectif (50) ;
dans lequel au moins une des N-1 faces exposées (18) du marqueur (10, 40) est identifiable, au moyen d'un algorithme de reconnaissance d'images, dans une image du marqueur (10, 40) capturée par un dispositif de capture d'images numériques.

15. Système (10, 40) selon la revendication 14, dans lequel le corps principal (12) a une forme choisie parmi un octaèdre, un dodécaèdre, un icosaèdre, de préférence un dodécaèdre.

FIG. 1

FIG. 2

FIG. 3

FIG. 4

FIG. 5

FIG. 6

FIG. 7

FIG. 8

FIG. 9

FIG. 10

500

```
                              ┌─────────┐
                              │  Start  │
                              └─────────┘
                                   │
```

501

┌ ─ ─ ─ ─ ─ ─ ─ ─ ─ ─ ─ ─ ─ ─ ┐          502
╎ Capture an uncalibrated image of ╎   ┌──────────────────────────────────┐
╎ calibration reference            ╎- ->│     Acquire an uncalibrated image      │
└ ─ ─ ─ ─ ─ ─ ─ ─ ─ ─ ─ ─ ─ ─ ┘   └──────────────────────────────────┘
                                                      │
                                         ┌──────────────────────────────────┐   504
                                         │ Define absolute calibrated reference system │
                                         │              (x, y, z)             │
503                                      └──────────────────────────────────┘
┌ ─ ─ ─ ─ ─ ─ ─ ─ ─ ─ ─ ─ ─ ─ ┐                      │                      506
╎ Capture a first calibrated image of the ╎   ┌──────────────────────────────────┐
╎ plurality of markers in the first plane (x, z) ╎- ->│ Acquire a first calibrated image of plurality of │
└ ─ ─ ─ ─ ─ ─ ─ ─ ─ ─ ─ ─ ─ ─ ┘          │              markers             │
┌ ─ ─ ─ ─ ─ ─ ─ ─ ─ ─ ─ ─ ─ ─ ┐          └──────────────────────────────────┘
╎ Capture a second calibrated image of the ╎                 │                      508
╎ plurality of markers in the second plane ╎   ┌──────────────────────────────────┐
╎ (y, z) perpendicular to the first plane (y, z) ╎- ->│ Acquire a second calibrated image of plurality │
└ ─ ─ ─ ─ ─ ─ ─ ─ ─ ─ ─ ─ ─ ─ ┘          │            of markers            │
                                         └──────────────────────────────────┘
               505                                    │                      510
                                         ┌──────────────────────────────────┐
                                         │ Determine a first initial positions $(X_{i1}, Z_{i1})$ of │
                                         │ contact points of markers in the first image │
                                         └──────────────────────────────────┘
                                                      │                      512
                                         ┌──────────────────────────────────┐
                                         │ Determine a second initial positions $(Y_{i2}, Z_{i2})$ of │
                                         │ contact points of markers in the second image │
                                         └──────────────────────────────────┘
                                                      │                      514
                                         ┌──────────────────────────────────┐
                                         │ Obtain positions $(X_i, Y_i, Z_i)$ of contact points of │
                                         │              markers             │
                                         └──────────────────────────────────┘
                                                      │                      516
                                         ┌──────────────────────────────────┐
                                         │ Fit the positions $(X_i, Y_i, Z_i)$ of contact points of │
                                         │ markers with a three-dimensional curve, │
                                         │ obtaining a reconstructed curve RC of the │
                                         │ anatomical structure of the subject │
                                         └──────────────────────────────────┘
                                                      │                      518
                                         ┌──────────────────────────────────┐
                                         │      Estimate parameters of interest     │
                                         └──────────────────────────────────┘
                                                      │                      520
                                         ┌──────────────────────────────────┐
                                         │       Display reconstructed curve RC      │
                                         └──────────────────────────────────┘
                                                      │                      522
                                         ┌──────────────────────────────────┐
                                         │               Store data              │
                                         └──────────────────────────────────┘
                                                      │
                                              ┌─────────┐
                                              │   End   │
                                              └─────────┘

FIG. 11

FIG. 12

510

524

| Recognize glyph |
|---|

526

| Define first relative reference system $(w_1, w_2, w_3)$ |
|---|

528

| Determine in the first calibrated image the position of center of symmetry $C_m$ of the main body of the marker |
|---|

530

| Determine in the first calibrated image the position of lower vertex $V_{low}$ of the marker |
|---|

532

| Determine in the first calibrated image the first initial position $(X_{i1}, Z_{i1})$ of the contact point of the marker at known distance from the center of symmetry $C_m$ of the main body of the marker along axis v connecting $C_m$ and lower vertex $V_{low}$ |
|---|

FIG. 13

FIG. 14

FIG. 15

FIG. 16

FIG. 17

**EP 4 061 214 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3225155 A1
- WO 2011149182 A2
- US 2003181830 A1

**Non-patent literature cited in the description**

- **D'AMICO M** ; **KINEL E** ; **RONCOLETTA P**. Normative 3D opto-electronic stereophotogrammetric posture and spine morphology data in young healthy adult population. *PLoS ONE*, 2017, vol. 12 (6), e0179619, https://doi.org/10.1371/journal.pone.0179619